# EUROPEAN PATENT APPLICATION

(11) **EP 4 750 286 A1**
(43) Date of publication of application: **27.05.2026**
(21) Application number: 25216533.7
(22) Date of filing: 18.11.2025
(51) Int. Cl.: H10K 50/12, H10K 85/60

(54) **ORGANIC COMPOUND AND ORGANIC LIGHT-EMITTING ELEMENT**

(30) Priority: 26.11.2024 JP 2024205450; 20.08.2025 JP 2025137179
(71) Applicant: Canon Kabushiki Kaisha, Tokyo, 146-8501 (JP)
(72) Inventor: NAKATANI, Erika, Tokyo, 146-8501 (JP); NISHIDE, Yosuke, Tokyo, 146-8501 (JP); KIKUCHI, Takayoshi, Tokyo, 146-8501 (JP); IWAWAKI, Hironobu, Tokyo, 146-8501 (JP); YAMADA, Naoki, Tokyo, 146-8501 (JP)
(74) Representative: TBK

(57) **Abstract**

An organic compound represented by formula [1]: 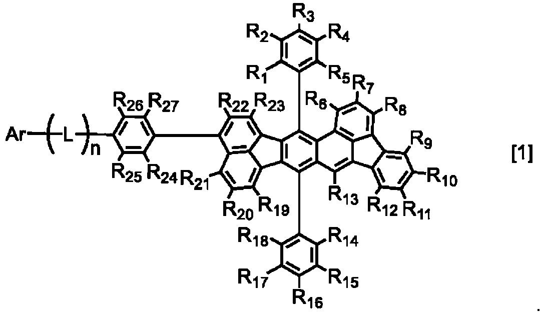 R₁ to R₂₇ denote a hydrogen atom or a substituent. Ar denotes a substituted or unsubstituted aryl group. n denotes an integer of 1 or more and 5 or less. L denotes any one of multiple structural formulas or a combination thereof. When n is 2 or more, a plurality of Ls may be the same or different.

## Description

### TECHNICAL FIELD

The present disclosure relates to an organic compound and an organic light-emitting element using the organic compound.

### BACKGROUND

An organic light-emitting element (hereinafter sometimes referred to as an "organic electroluminescent element" or an "organic EL element") is an electronic element including a pair of electrodes and an organic compound layer disposed between the electrodes. Injecting electrons and holes from the pair of electrodes generates excitons of a light-emitting organic compound in the organic compound layer, and the organic light-emitting element emits light when the excitons return to the ground state.

The recent progress of an organic light-emitting element is remarkable, and examples thereof include a low drive voltage, various emission wavelengths, high-speed responsivity, and a decrease in thickness and weight of a light-emitting device.

With regard to an increase in efficiency of a light-emitting element, an element using a higher efficiency material, such as a phosphorescent material or a delayed fluorescent material, has been reported.

As a compound created so far, the following compound 1-a is described in Japanese Patent Laid-Open No. 2010-270103 (PTL 1). Furthermore, International Publication No. 2017/146192 (PTL 2) discloses the following compound 2-a. These compounds have acenaphtho[1,2-k]benzo[e]acephenanthrene as a basic skeleton.

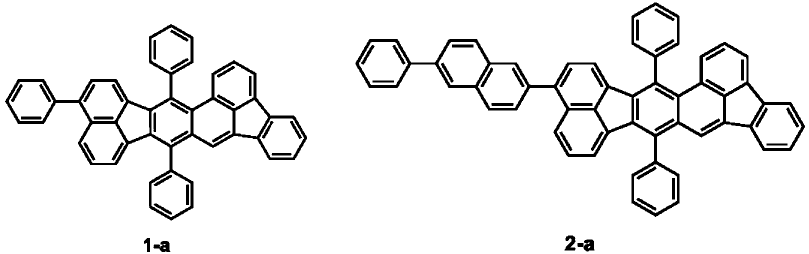

However, since the compound 1-a has a structure in which the compounds are difficult to be horizontally oriented, there is room for improvement from the perspective of light emission efficiency. Furthermore, since the compound 2-a has a long conjugation length, there is room for improvement from the perspective of color purity.

### SUMMARY

The present embodiment has been made in view of the above disadvantages and is directed to provide an organic compound with high light emission efficiency and color purity.

The present disclosure in its first aspect provides an organic compound as specified in claim 1. Optional features are specified in claims 2 to 9.

The present disclosure in its second aspect provides an organic light-emitting element as specified in claim 10. Optional features are specified in claims 11 to 13.

The present disclosure in its third aspect provides a display apparatus as specified in claim 14.

The present disclosure in its fourth aspect provides a photoelectric conversion apparatus as specified in claim 15.

The present disclosure in its fifth aspect provides an image display apparatus as specified in claim 16.

The present disclosure in its sixth aspect provides electronic equipment as specified in claim 17.

The present disclosure in its seventh aspect provides a wearable device as specified in claim 18.

The present disclosure in its eighth aspect provides a lighting apparatus as specified in claim 19.

The present disclosure in its ninth aspect provides a movable body as specified in claim 20.

Features of the present disclosure will become apparent from the following description of embodiments with reference to the attached drawings. The following description of embodiments is described by way of example.

### BRIEF DESCRIPTION OF THE DRAWINGS

Fig. 1A is a schematic cross-sectional view of an example of a pixel of a display apparatus according to an embodiment of the present embodiment.
Fig. 1B is a schematic cross-sectional view of an example of a display apparatus including an organic EL element according to an embodiment of the present embodiment.
Fig. 2 is a schematic view of an example of a display apparatus according to an embodiment of the present embodiment.
Fig. 3A is a schematic view of an example of an imaging apparatus according to an embodiment of the present embodiment.
Fig. 3B is a schematic view of an example of electronic equipment according to an embodiment of the present embodiment.
Fig. 4A is a schematic view of an example of a display apparatus according to an embodiment of the present embodiment.
Fig. 4B is a schematic view of an example of a foldable display apparatus.
Fig. 5A is a schematic view of an example of a lighting apparatus according to an embodiment of the present embodiment.
Fig. 5B is a schematic view of an example of an automobile having a vehicle lamp according to an embodiment of the present embodiment.
Fig. 5C is a schematic view of a steering wheel, a display unit, and the like of an automobile according to an embodiment of the present embodiment.
Fig. 6A is a schematic view of an example of a wearable device according to an embodiment of the present embodiment.
Fig. 6B is a schematic view of an example of a wearable device according to an embodiment of the present embodiment, which includes an imaging apparatus.
Fig. 7A is a schematic view of an example of an image-forming apparatus according to an embodiment of the present embodiment.
Fig. 7B is a schematic view of an example of an exposure light source of an image-forming apparatus according to an embodiment of the present embodiment.
Fig. 7C is a schematic view of an example of an exposure light source of an image-forming apparatus according to an embodiment of the present embodiment.
Fig. 8 is a view of a transition dipole moment direction and a light emission direction of an organic compound.
Fig. 9 is a view of HOMO orbital distributions of an exemplary compound A1 and a comparative compound 2-a.
Fig. 10 is a view of HOMO orbital distributions of exemplary compounds B24, B42, and A26.

### DESCRIPTION OF THE EMBODIMENTS

In the present specification, a halogen atom is, for example, but not limited to, fluorine, chlorine, bromine, iodine, astatine, tennessine, or the like.

The alkyl group may be an alkyl group having 1 or more and 20 or less carbon atoms or an alkyl group having 1 or more and 10 or less carbon atoms. Specific examples thereof include, but are not limited to, a methyl group, an ethyl group, a n-propyl group, an isopropyl group, a n-butyl group, a tert-butyl group, a sec-butyl group, an octyl group, a cyclohexyl group, a tert-pentyl group, a 3-methylpentan-3-yl group, a 1-adamantyl group, and a 2-adamantyl group.

The alkoxy group may be an alkoxy group having 1 or more and 20 or less carbon atoms or an alkoxy group having 1 or more and 10 or less carbon atoms. Specific examples thereof include, but are not limited to, a methoxy group, an ethoxy group, a propoxy group, an isopropoxy group, a tert-butoxy group, a 2-ethyl-octyloxy group, and a benzyloxy group.

A silyl group is a group in which a silicon atom has a hydrogen atom or a substituent. The substituent may be a substituted or unsubstituted alkyl group or a substituted or unsubstituted aryl group. The substituted or unsubstituted alkyl group of the silicon atom may be a substituted or unsubstituted alkyl group having 1 or more and 4 or less carbon atoms. The substituted or unsubstituted aryl group of the silicon atom may be a substituted or unsubstituted aryl group having 6 or more and 10 or less carbon atoms. The silyl group may be a trialkylsilyl group or a triarylsilyl group. Specific examples thereof include, but are not limited to, a trimethylsilyl group and a triphenylsilyl group.

The aryl group may be an aryl group having 6 or more and 20 or less carbon atoms, an aryl group having 6 or more and 18 or less carbon atoms, or an aryl group having 6 or more and 12 or less carbon atoms. Specific examples thereof include, but are not limited to, a phenyl group, a biphenyl group, a naphthyl group, a phenanthrenyl group, a triphenylenyl group, an indenyl group, a terphenyl group, a fluorenyl group, a pyrenyl group, an anthranyl group, a perylenyl group, a chrysenyl group, and a fluoranthenyl group.

The heteroaryl group may be a heteroaryl group having 3 or more and 24 or less carbon atoms, a heteroaryl group having 3 or more and 18 or less carbon atoms, or a heteroaryl group having 3 or more and 12 or less carbon atoms. Specific examples thereof include, but are not limited to, a pyridyl group, a pyrimidyl group, a pyrazyl group, a triazyl group, a benzofuranyl group, a benzothiophenyl group, a dibenzofuranyl group, a dibenzothiophenyl group, an oxazolyl group, an oxadiazolyl group, a thiazolyl group, a thiadiazolyl group, a carbazolyl group, an acridinyl group, and a phenanthrolyl group.

The amino group may be a substituted amino group substituted with an alkyl group or an aryl group or may be a substituted amino group substituted with an alkyl group having 1 or more and 4 or less carbon atoms or an aryl group having 6 or more and 12 or less carbon atoms. Specific examples thereof include, but are not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-tert-butylphenyl)amino group, an N-phenyl-N-(4-trifluoromethylphenyl)amino group, and an N-piperidyl group.

Specific examples of the aryloxy group include, but are not limited to, a phenoxy group.

Specific examples of the heteroaryloxy group include, but are not limited to, a thienyloxy group.

A substituent that the alkyl group, the alkoxy group, the amino group, the aryloxy group, the silyl group, the aryl group, the heteroaryl group, and the heteroaryloxy group may further have is, for example, but not limited to, deuterium, an alkyl group, such as a methyl group, an ethyl group, a n-propyl group, an isopropyl group, a n-butyl group, or a tert-butyl group, an aralkyl group, such as a benzyl group, an aryl group, such as a phenyl group or a biphenyl group, a heterocyclic group, such as a pyridyl group or a pyrrolyl group, an amino group, such as a dimethylamino group, a diethylamino group, a dibenzylamino group, a diphenylamino group, or a ditolylamino group, an alkoxy group, such as a methoxy group, an ethoxy group, or a propoxy group, an aryloxy group, such as a phenoxy group, a halogen atom, such as fluorine, chlorine, bromine, or iodine, a cyano group, or the like.

In the present specification, the basic skeleton refers to an acenaphtho[1,2-k]benzo[e]acephenanthrene skeleton.

### (1) Organic Compound

The organic compounds according to the present embodiment are represented by the general formula [1].

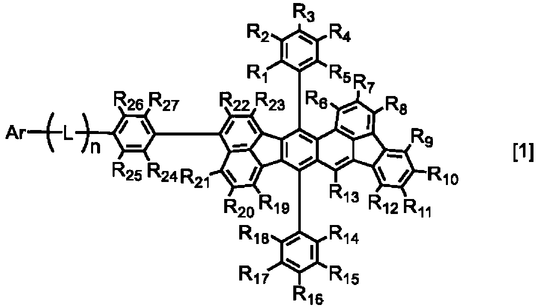

### <<R₁ to R₂₇>>

In the general formula [1], R₁ to R₂₇ are each independently selected from the group consisting of a hydrogen atom, a deuterium atom, a substituted or unsubstituted alkyl group, a substituted or unsubstituted alkoxy group, a substituted or unsubstituted aryl group, a substituted or unsubstituted aryloxy group, a substituted or unsubstituted heteroaryl group, a substituted or unsubstituted heteroaryloxy group, a substituted or unsubstituted amino group, and a cyano group.

R₁ to R₂₇ may be each independently selected from the group consisting of a hydrogen atom, a deuterium atom, a substituted or unsubstituted alkyl group, a substituted or unsubstituted aryl group, and a substituted or unsubstituted heteroaryl group. R₁ to R₂₇ may be each independently selected from the group consisting of a hydrogen atom, a deuterium atom, a substituted or unsubstituted alkyl group having 1 or more and 6 or less carbon atoms, and a substituted or unsubstituted aryl group having 6 or more and 12 or less carbon atoms. R₁ to R₂₇ may be each independently selected from the group consisting of a hydrogen atom, a substituted or unsubstituted alkyl group having 1 or more and 6 or less carbon atoms, and a phenyl group. More specifically, R₁ to R₂₇ may be each independently selected from the group consisting of a hydrogen atom, a deuterium atom, a methyl group, a CD₃ group, an ethyl group, an isopropyl group, a tert-butyl group, C(CH₃)₂(C₂H₅), C(CH₃)(C₂H₅)₂, a phenyl group, a phenyl group having a cyclohexyl group, and a biphenyl group. R₁ to R₂₇ may be each independently selected from the group consisting of a hydrogen atom, a deuterium atom, a methyl group, a CD₃ group, an isopropyl group, a tert-butyl group, C(CH₃)₂(C₂H₅), C(CH₃)(C₂H₅)₂, and a phenyl group. R₁ to R₂₇ may be each independently selected from the group consisting of a hydrogen atom, a methyl group, an isopropyl group, a tert-butyl group, C(CH₃)(C₂H₅)₂, and a phenyl group.

At least one of R₁ to R₂₇ may be other than a hydrogen atom. At least one of R₁, R₃, R₅, R₁₁, R₁₄, R₁₆, R₁₈, R₂₄, and R₂₇ may be other than a hydrogen atom, and at least one of R₁, R₃, R₅, R₁₄, R₁₆, R₁₈, R₂₄, and R₂₇ may be other than a hydrogen atom. In one aspect of the present embodiment, at least one of R₁, R₅, R₁₄, and R₁₈ may denote a substituent other than a hydrogen atom, and in such a case, the substituent other than a hydrogen atom may be a substituted or unsubstituted alkyl group or a substituted or unsubstituted aryl group, and is preferably an alkyl group having 1 or more and 4 or less carbon atoms or an aryl group having 6 or more and 10 or less carbon atoms from the perspective of the molecular weight. More specifically, a methyl group or a phenyl group is preferred.

Furthermore, in one aspect of the present embodiment, at least one of R₃ and R₁₆ may denote a substituent other than a hydrogen atom, and in such a case, the substituent other than a hydrogen atom may be a substituted or unsubstituted alkyl group. From the perspective of the molecular weight, an alkyl group having 1 or more and 6 or less carbon atoms is preferred. More specifically, a methyl group, a tert-butyl group, or C(CH₃)(C₂H₅)₂ is preferred.

Furthermore, in one aspect of the present embodiment, at least one of R₂₄ and R₂₇ may denote a hydrogen atom, and in such a case, the substituent other than a hydrogen atom may be a substituted or unsubstituted alkyl group and is, from the perspective of the molecular weight, preferably an alkyl group having 1 or more and 4 or less carbon atom. More specifically, a methyl group is preferred.

Furthermore, at least one of a pair of R₂₁ and R₂₄ and a pair of R₂₂ and R₂₇ may be bonded to each other to form a ring. The ring is preferably represented by the general formula [1a] or [1b].

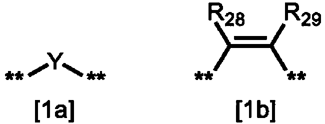

In the general formula [1a], Y denotes a chalcogen atom, preferably an oxygen atom or a sulfur atom.

In the general formula [1b], R₂₈ and R₂₉ are each independently selected from the group consisting of a hydrogen atom, a deuterium atom, a halogen atom, a substituted or unsubstituted alkyl group, a substituted or unsubstituted alkoxy group, a substituted or unsubstituted aryl group, a substituted or unsubstituted aryloxy group, a substituted or unsubstituted heteroaryl group, a substituted or unsubstituted heteroaryloxy group, a substituted or unsubstituted amino group, and a cyano group. R₂₈ and R₂₉ may be bonded to each other to form a ring.

R₂₈ and R₂₉ are preferably each independently selected from the group consisting of a hydrogen atom, a deuterium atom, a halogen atom, and a substituted or unsubstituted alkyl group, more preferably each independently selected from a hydrogen atom, a deuterium atom, a fluorine atom, and a substituted or unsubstituted alkyl group having 1 or more and 4 or less carbon atoms, still more preferably each independently selected from a hydrogen atom, a deuterium atom, and a substituted or unsubstituted alkyl group having 1 or more and 4 or less carbon atoms.

In the general formulae [1a] and [1b], ** denotes a binding position to the general formula [1].

The ring formed by combining at least one of a pair of R₂₁ and R₂₄ and a pair of R₂₂ and R₂₇ may be an aromatic hydrocarbon ring having 5 or more and 10 or less carbon atoms or a heteroaromatic ring having 4 or more and 10 or less carbon atoms. More specifically, the ring preferably forms a 6-membered ring and preferably forms a benzene ring. The same applies to the ring formed by combining R₂₈ and R₂₉.

### <<Ar>>

In the general formula [1], Ar denotes a substituted or unsubstituted aryl group. From the perspective of the molecular weight, Ar preferably denotes a substituted or unsubstituted aryl group having 6 or more and 20 or less carbon atoms, more preferably a substituted or unsubstituted aryl group having 6 or more and 12 or less carbon atoms, still more preferably an aryl group having 6 or more and 10 or less carbon atoms. More specifically, Ar may denote a phenyl group, a naphthyl group, a biphenyl group, a triphenylene group, or a phenanthrene group. Preferred are a phenyl group, a naphthyl group, and a biphenyl group, each of which may have a tert-butyl group as a substituent. More preferably, Ar may denote a phenyl group or a naphthyl group.

When Ar has a substituent, the substituent may be a deuterium atom, a substituted or unsubstituted alkyl group, or a substituted or unsubstituted aryl group. From the perspective of the molecular weight, a deuterium atom or an alkyl group having 1 or more and 6 or less carbon atoms is preferred, an alkyl group having 1 or more and 6 or less carbon atoms is more preferred, and an alkyl group having 1 or more and 4 or less carbon atoms is still more preferred. More specifically, the substituent may be a deuterium atom, a phenyl group, a naphthyl group, a biphenyl group, a triphenylene group, or a phenanthrene group, preferably a methyl group, a tert-butyl group, C(CH₃)(C₂H₅)₂, a cyclopentyl group, a cyclohexyl group, or an adamantyl group. More preferred is a tert-butyl group or a cyclohexyl group. Still more preferred is a tert-butyl group.

Furthermore, when Ar has a substituent, the substituent is preferably introduced into an ortho position with respect to L in the general formula [1].

### <<L>>

In the general formula [1], L denotes any one of the general formulae [2] to [7] or a combination thereof. For example, when L denotes a combination of the general formulae [2] and [3], n described later is 2.

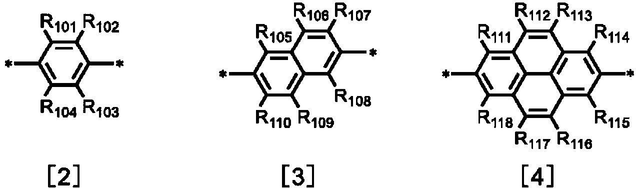

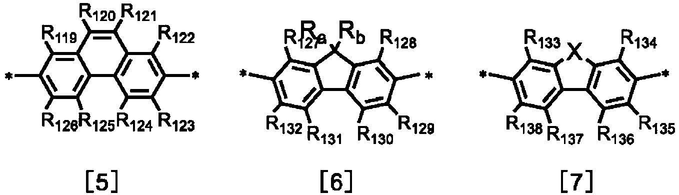

In the general formulae [2] to [7], R₁₀₁ to R₁₃₈, Rₐ, and R_{b} are each independently selected from a hydrogen atom, a substituted or unsubstituted alkyl group, and a substituted or unsubstituted aryl group. X denotes an oxygen atom or a sulfur atom. * denotes a binding position.

Rₐ and R_{b} may denote an alkyl group having 1 or more and 4 or less carbon atoms or an aryl group having 6 or more and 10 or less carbon atoms and, more specifically, may denote a methyl group or a phenyl group. From the perspective of the molecular weight, a methyl group is more preferred.

Rₐ and R_{b} may denote bonded to each other to form a ring. The ring may be a substituted or unsubstituted aromatic hydrocarbon ring or a substituted or unsubstituted heteroaromatic ring and may be a substituted or unsubstituted aromatic hydrocarbon ring. More specifically, Rₐ and R_{b} may be bonded to each other to form a fluorene skeleton. In other words, when L is represented by the general formula [6], the general formula [6] may be a spirofluorene skeleton.

L may be represented by the general formulae [2] to [7], a plurality of the general formulae [2], or a combination of the general formula [2] and the general formula [3], may be the general formulae [2] to [7] or a plurality of the general formulae [2], or may be the general formula [2], [3], or [4], or a plurality of the general formulae [2]. In such a case, X may denote an oxygen atom.

Furthermore, R₁₀₁ to R₁₃₈ may denote a hydrogen atom, a deuterium atom, or a substituted or unsubstituted alkyl group having 1 or more and 4 or less carbon atoms, and may denote a hydrogen atom or a substituted or unsubstituted alkyl group having 1 or more and 2 or less carbon atoms. Specific examples thereof may include a hydrogen atom, a methyl group, a CD₃ group, and an ethyl group, and may include a hydrogen atom, a methyl group, and an ethyl group.

### <<n>>

In the general formula [1], n denotes an integer of 1 or more and 5 or less. The larger the value of n, the better the molecular orientation property, and the smaller the value of n, the higher the sublimability. Thus, n is preferably 1 or more and 3 or less.

Next, a method for synthesizing the organic compounds according to the present embodiment will be described. The organic compounds according to the present embodiment are synthesized, for example, according to the following reaction scheme.

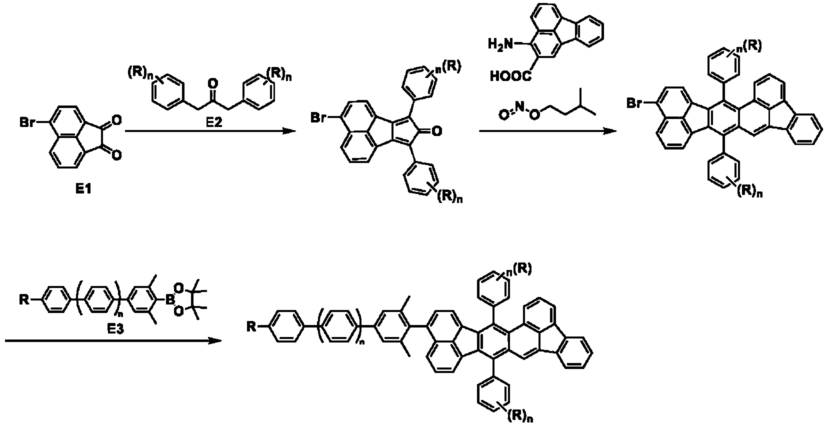

As shown in the above synthesis scheme, the organic compounds according to the present embodiment are synthesized using compounds shown in the following (a) to (c) as raw materials.
(a) An acenaphthene quinone derivative (E1)
(b) A dibenzyl ketone derivative (E2)
(c) A long-chain aromatic hydrocarbon derivative (E3)

Here, by appropriately introducing a substituent into the compounds (a) to (c), any one of R₁ to R₂₇ in the formula (1) is substituted from a hydrogen atom to a predetermined group other than the hydrogen atom. In the above synthesis scheme, various organic compounds can be synthesized by varying E1 to E3, respectively. However, the synthesis method is not limited thereto.

Next, the organic compounds according to the present embodiment will be described. The organic compounds according to the present embodiment have the following configuration and therefore have high light emission efficiency and color purity. In the present specification, the emission wavelength of blue light emission with high color purity refers to light emission in which an emission spectrum in a dilute toluene solution has a peak in the range of 430 nm or more less than 460 nm.
(1-1) Since the basic skeleton has a plurality of aryl groups in the major-axis direction, the horizontal orientation property is improved.
(1-2) When benzene is directly bonded in the major-axis direction of the basic skeleton, π conjugation is less likely to extend.

These will be described in detail below.

(1-1) Since the basic skeleton has a plurality of aryl groups in the major-axis direction, the horizontal orientation property is improved.

On the basis of intensive study results, the present inventors have found that when the basic skeleton has a plurality of aryl groups in the major-axis direction, the horizontal orientation property can be improved, and the effect of improving the light emission efficiency can be obtained.

The horizontal orientation property of an organic compound can be represented by a statistical parameter called a Pz value. When the Pz value is 0, the organic compound is disposed parallel to the substrate, and when the Pz value is 1, the organic compound is disposed perpendicular to the substrate. Thus, a smaller Pz value is preferred because the organic compound has an improved horizontal orientation property.

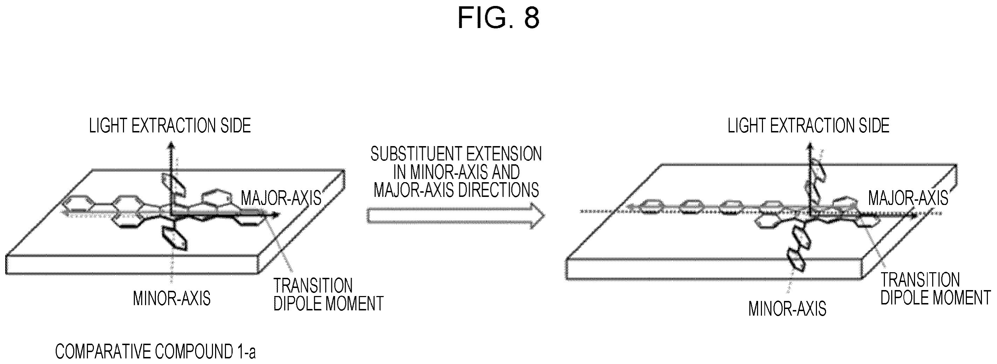

Fig. 8 illustrates the transition dipole moment direction and the light emission direction of an organic compound according to the present embodiment and a comparative compound. As illustrated in Fig. 8, light emitted from the organic compound is extracted in a direction approximately perpendicular to a transition dipole moment (a factor determining the direction and intensity of an electric field of light emission) in the molecule. In the organic compound according to the present embodiment, the transition dipole moment in the molecule is in the major-axis direction of the basic skeleton. Thus, when the organic compound according to the present embodiment is horizontally oriented with respect to the substrate, the transition dipole moment in the molecule can also be horizontally oriented with respect to the substrate. Consequently, light emitted from the organic compound can be extracted in a direction approximately perpendicular to the substrate. For the above reasons, an organic compound with high light emission efficiency can be obtained by increasing the horizontal orientation property of the organic compound according to the present embodiment.

To clarify the relationship between the horizontal orientation property and the light emission efficiency of the organic compound, the Pz values and the light emission efficiencies of the organic compounds according to the present embodiment and comparative compounds were measured and compared. Table 1 shows the results. The Pz values are values measured using a "molecular orientation characteristic measuring apparatus C14234-01" manufactured by Hamamatsu Photonics K.K. for a film having the same configuration as that of a light-emitting layer, which was formed on silica glass by a vacuum deposition method. The light emission efficiencies are relative values when the light emission efficiency of Comparative Example 2 is taken as 1.0.

**Table 1**

| | Molecular structure | Pz value | Light emission efficiency |
|---|---|---|---|
| Element of present disclosure 1 | 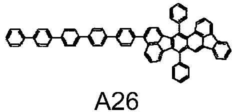 | 0.03 | 1.4 |
| Element of present disclosure 2 | 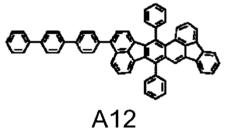 | 0.05 | 1.3 |
| Comparative element 1 | 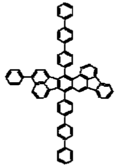 Comparative compound 1-b | 0.10 | 1.1 |
| Comparative element 2 | 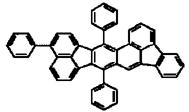 Comparative compound 1-a | 0.11 | 1.0 |

From Table 1, the Pz values of the organic compounds according to the present embodiment were 0.03 and 0.05, which are smaller than the Pz values of comparative compounds 1-a and 1-b. It is thought that the Pz values were smaller because the organic compounds according to the present embodiment have a substituent in the major-axis direction of the basic skeleton. It is thought that the smaller Pz values resulted in the improved horizontal orientation property and improved light extraction efficiency of the organic compounds and resulted in the high light emission efficiency.

On the other hand, the comparative compound 1-a has a structure in which one benzene is bonded in each of the major-axis direction and the minor-axis direction of the organic compound. The comparative compound 1-b has a structure in which a plurality of benzenes, which are aryl groups, are bonded in the minor-axis direction of the organic compound, but only one benzene group is bonded in the major-axis direction. In these compounds, the organic compounds do not have a plurality of aryl groups in the major-axis direction and have a larger Pz value than the organic compounds according to the present embodiment. Thus, the organic compounds according to the present embodiment have a higher horizontal orientation property and therefore higher light emission efficiency than the comparative compounds.

Thus, it has been found that the organic compounds according to the present embodiment have a small Pz value by bonding a plurality of aryl groups in the major-axis direction of the organic compounds, and therefore the horizontal orientation property of the basic skeleton is improved.

(1-2) When benzene is directly bonded in the major-axis direction of the basic skeleton, π conjugation is less likely to extend.

In the organic compounds according to the present embodiment, when benzene is directly bonded in the major-axis direction of the basic skeleton, π conjugation is less likely to extend. Consequently, the organic compounds according to the present embodiment can provide blue light emission with high color purity.

As described above, the organic compounds according to the present embodiment are substituted with a plurality of aryl groups in the major-axis direction, which is the transition dipole moment direction of the basic skeleton, to reduce the Pz value, which also leads to extension of π conjugation. Since extension of π conjugation causes an increase in the emission wavelength of an organic compound, extension of π conjugation needs to be reduced to obtain blue light emission with high color purity. Thus, the present inventors have found that when benzene is directly bonded to the basic skeleton in its major-axis direction, the horizontal orientation property of the basic skeleton can be improved while the extension of the π conjugation is reduced.

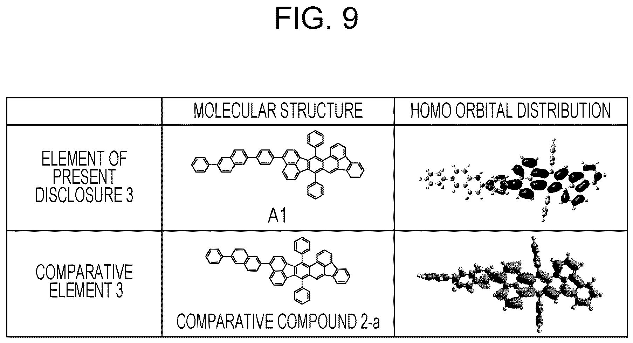

Table 2 shows the emission wavelengths and Pz values of an organic compound according to the present embodiment and the comparative compound 2-a. Fig. 9 shows the HOMO distributions of these compounds.

**Table 2**

| | Molecular structure | Emission wavelength/nm | Pz value |
|---|---|---|---|
| Element of present disclosure 3 | 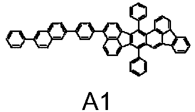 | 450 | 0.07 |
| Comparative element 3 | 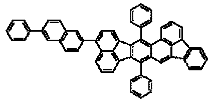 Comparative compound 2-a | 461 | 0.08 |

Table 2 shows that A1, which is an organic compound according to the present embodiment, has a Pz value equal to or higher than that of the comparative compound 2-a and emits blue light having a shorter wavelength than the comparative compound 2-a. This is probably because extension of π conjugation can be reduced by direct bonding of benzene to the basic skeleton. For example, referring to Fig. 9, in the exemplary compound A1, the π conjugation extends over the basic skeleton and the phenyl groups directly bonded to the basic skeleton, and the π conjugation is broken between the phenyl groups and the naphthyl group. On the other hand, in the comparative compound 2-a, the π conjugation extends to the basic skeleton and the naphthyl group directly bonded to the basic skeleton. Thus, the organic compound according to the present embodiment can suppress the extension of π conjugation as compared with the comparative compound 2-a. Consequently, the emission wavelength of the organic compound according to the present embodiment is shorter by 11 nm than that of the comparative compound 2-a. Thus, the organic compound according to the present embodiment can emit blue light with high color purity.

Since a substituent bonded to a substituent directly bonded to the basic skeleton is not involved in the extension of π conjugation, the substituent may be a fused polycyclic substituent.

The above electron orbital distribution was visualized by molecular orbital calculation. The density functional theory (DFT), which is currently widely used, was used as a calculation method of the molecular orbital calculation method. B3LYP was used as a functional, and 6-31G* was used as a basis function. The molecular orbital calculation method was performed by currently widely used Gaussian 09 (Gaussian 09, Revision C. 01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. lyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010.). The molecular orbital calculation in the present specification uses the same method.

For the above reasons, the organic compounds according to the present embodiment have a plurality of aryl groups in the major-axis direction with respect to the basic skeleton, and therefore the horizontal orientation property is improved. Furthermore, when the aryl group directly bonded to the basic skeleton is a phenyl group, the extension of π conjugation can be reduced. Thus, the organic compounds according to the present embodiment have high color purity and light emission efficiency.

Furthermore, the organic compounds according to the present embodiment preferably have the following configuration.
(1-3) At least one of R₂₄ and R₂₇ denotes a substituted or unsubstituted alkyl group.
(1-4) At least one of R₁, R₅, R₁₄, and R₁₈ denotes a substituted or unsubstituted alkyl group or a substituted or unsubstituted aryl group.
(1-5) At least one of Ar, R₃, and R₁₆ has a secondary alkyl group or a tertiary alkyl group.

These configurations will be described below.

(1-3) At least one of R₂₄ and R₂₇ denotes a substituted or unsubstituted alkyl group.

In the organic compounds according to the present embodiment, at least one of R₂₄ and R₂₇ preferably denotes a substituted or unsubstituted alkyl group. This configuration can further suppress the extension of π conjugation and further reduce the emission wavelength. Consequently, the organic compounds according to the present embodiment emit blue light with high color purity.

In the organic compounds according to the present embodiment, at least one of R₂₄ and R₂₇ preferably denotes an alkyl group having 1 or more and 6 or less carbon atoms, more preferably an alkyl group having 1 or more and 4 or less carbon atoms. More specifically, it may be a methyl group, a CD₃ group, an ethyl group, an isopropyl group, or a tert-butyl group, may be a methyl group or a CD₃ group, and may be a methyl group. R₂₄ and R₂₇ preferably denote a substituted or unsubstituted alkyl group.

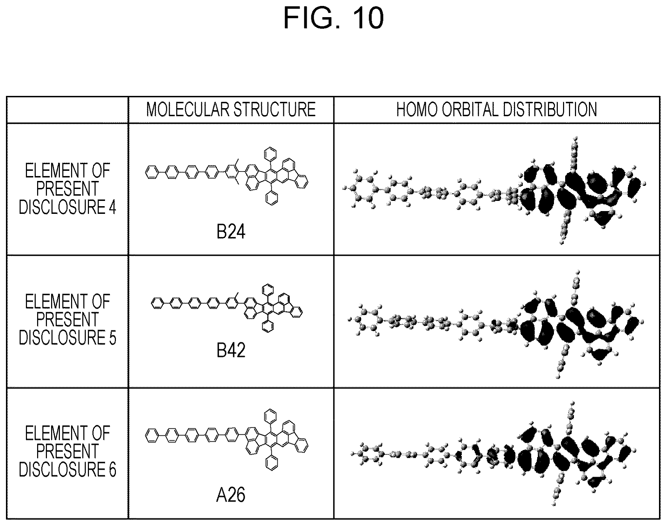

Table 3 shows the emission wavelengths and Pz values of exemplary compounds A26, B42, and B24, which are the organic compounds according to the present embodiment. Fig. 10 shows the HOMO orbital distributions of these compounds.

**Table 3**

| | Molecular structure | Emission wavelength/nm | Pz value |
|---|---|---|---|
| Element of present disclosure 4 | 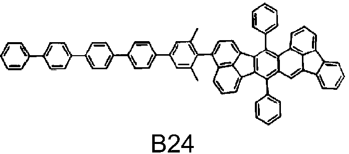 | 445 | 0.03 |
| Element of present disclosure 5 | 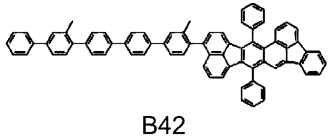 | 450 | 0.03 |
| Element of present disclosure 6 | 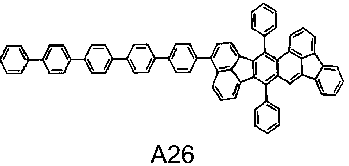 | 457 | 0.03 |

Table 3 shows that the exemplary compounds A26, B24, and B42, which are the organic compounds according to the present embodiment, have different emission wavelengths. Referring to the HOMO orbital distributions shown in Fig. 10, the HOMO orbital distributions of the exemplary compound B24 in which R₂₄ and R₂₇ denote methyl groups spread only to the basic skeleton, and the HOMO orbital distribution of the exemplary compound B42 in which R₂₇ denotes a methyl group spreads to the basic skeleton and the phenyl groups directly bonded to the basic skeleton. On the other hand, the HOMO orbital distribution of the exemplary compound A26 in which R₂₄ and R₂₇ are hydrogen atoms spread not only to the basic skeleton and the phenyl groups directly bonded to the basic skeleton but also to the phenyl group further bonded to the phenyl groups.

This is because when at least one of R₂₄ and R₂₇ denotes a substituted or unsubstituted alkyl group, the basic skeleton and the substituents bonded to the basic skeleton are arranged in a twisted manner, so that the HOMO orbital distribution is less likely to spread. Consequently, the organic compounds according to the present embodiment emit blue light having a shorter wavelength while maintaining the Pz value and therefore have higher color purity.

(1-4) At least one of R₁, R₅, R₁₄, and R₁₈ denotes a substituted or unsubstituted alkyl group or a substituted or unsubstituted aryl group.

In the organic compounds according to the present embodiment, at least one of R₁, R₅, R₁₄, and R₁₈ preferably denotes a substituted or unsubstituted alkyl group or a substituted or unsubstituted aryl group. This configuration can further suppress intermolecular stacking of organic compounds and therefore improve the sublimability. Furthermore, the suppression of the intermolecular stacking can also suppress concentration quenching and therefore improve the photoluminescence quantum yield (PLQY).

In particular, an improvement in sublimability is important in terms of increasing the purity of an organic compound. In general, when an organic compound is used for an organic light-emitting element, impurities are removed by sublimation purification. Thus, an organic compound with high sublimability can be purified by sublimation to produce a high-purity organic compound. Consequently, it is possible to further reduce element deterioration due to impurities during element driving, and it is therefore preferable that the sublimation margin temperature has a large value. Furthermore, an organic compound preferably has high sublimability because decomposition of the organic compound can be reduced during sublimation purification or vapor deposition. The sublimation margin temperature is a difference between the decomposition temperature of an organic compound and the sublimation temperature (temperature at which sublimation starts) of the organic compound. The decomposition temperature of an organic compound can be defined as the temperature at which the degree of vacuum deteriorates.

Table 4 shows the sublimation margin temperatures and the element durability ratios of C8, C5, C56, and B22, which are the organic compounds according to the present embodiment.

**Table 4**

| | Molecular structure | Sublimation margin temperature/°C | Element durability ratio |
|---|---|---|---|
| Element of present disclosure 7 | 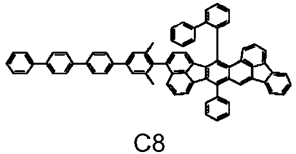 | 70 | 1.3 |
| Element of present disclosure 8 | 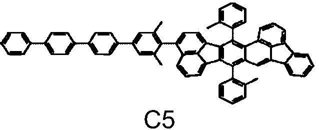 | 60 | 1.2 |
| Element of present disclosure 9 | 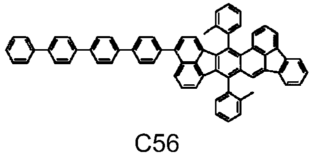 | 60 | 1.1 |
| Element of present disclosure 11 | 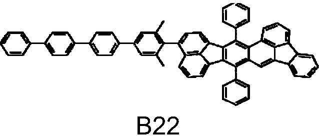 | 30 | 1.0 |

Table 4 shows that the exemplary compounds C8, C5, and C56 in which at least one of R₁, R₅, R₁₄, and R₁₈ denotes a substituted or unsubstituted alkyl group or a substituted or unsubstituted aryl group have a higher sublimation margin temperature than the exemplary compound B22 in which R₁, R₅, R₁₄, and R₁₈ denotes hydrogen atoms. Consequently, the exemplary compounds C8, C5, and C56 were more durable than the exemplary compound B22.

The basic skeleton of the organic compounds according to the present embodiment has a structure with high planarity and a structure that is easily subjected to intermolecular stacking. Thus, when at least one of R₁, R₅, R₁₄, and R₁₈ denotes a substituted or unsubstituted alkyl group or a substituted or unsubstituted aryl group, these substituents can sufficiently cover the basic skeleton. It is thought that this further enhances the effect of suppressing the intermolecular stacking, increases the sublimation margin temperature, and results in high element durability.

In the organic compounds according to the present embodiment, at least one of R₁, R₅, R₁₄, and R₁₈ preferably denotes a substituted or unsubstituted alkyl group because the Pz value is reduced. Using the above-described method, the Pz values of exemplary compounds C4 and C7 were determined to be 0.05 and 0.06, respectively, and it was found that the horizontal orientation property was improved when at least one of R₁, R₅, R₁₄, and R₁₈ denotes a substituted or unsubstituted alkyl group. It is thought that when at least one of R₁, R₅, R₁₄, and R₁₈ denotes a substituted or unsubstituted alkyl group, it more easily interacts with the basic skeleton, resulting in an improved horizontal orientation property of the organic compounds according to the present embodiment. For the above reasons, in the organic compounds according to the present embodiment, at least one of R₁, R₅, R₁₄, and R₁₈ preferably denotes a substituted or unsubstituted alkyl group.

(1-5) At least one of Ar, R₃, and R₁₆ has a secondary alkyl group or a tertiary alkyl group.

In the organic compounds according to the present embodiment, at least one of Ar, R₃, and R₁₆ has a secondary alkyl group or a tertiary alkyl group, can further suppress intermolecular stacking, and can further reduce the concentration quenching. Consequently, the organic compounds according to the present embodiment are organic compounds with high PLQY. The secondary alkyl group or the tertiary alkyl group may be an iso-propyl group or a tert-butyl group, and these alkyl groups are preferred due to their bulky structures.

The secondary alkyl group or the tertiary alkyl group may be bonded to Ar, R₃, and R₁₆ through an aryl group or a heteroaryl group. From the perspective of the molecular weight, at least one of Ar, R₃, or R₁₆ preferably denotes a secondary alkyl group or a tertiary alkyl group, more preferably an iso-propyl group or a tert-butyl group, still more preferably a tert-butyl group.

Table 5 shows the PLQYs and Pz values of D9 and B19, which are the organic compounds according to the present embodiment. The PLQYs were measured using an "absolute PL quantum yield measurement system" manufactured by Hamamatsu Photonics K.K. for a 30-nm thin film, which was formed on silica glass by a vacuum deposition method at a ratio of 99% by weight of one type of host material and 1% by weight of a guest material (an organic compound according to the present embodiment).

**Table 5**

| | Molecular structure | PLQY/% | Pz value |
|---|---|---|---|
| Element of present disclosure 12 | 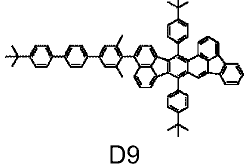 | 79 | 0.04 |
| Element of present disclosure 13 | 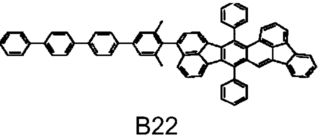 | 73 | 0.06 |

Table 5 shows that an exemplary compound D9 having tert-butyl groups in which Ar, R₃, and R₁₆ denote tertiary alkyl groups had a higher PLQY than the exemplary compound B22 in which Ar, R₃, and R₁₆ denote hydrogen atoms. This is probably because Ar, R₃, and R₁₆ have a bulky alkyl group and can therefore suppress the intermolecular stacking. Thus, the organic compounds according to the present embodiment have a high PLQY because at least one of Ar, R₃, and R₁₆ has a secondary alkyl group or a tertiary alkyl group.

Specific examples of the organic compounds according to the present embodiment are described below. However, the present embodiment is not limited thereto. It should be noted that structural isomers of the following compounds are also included as exemplary compounds.

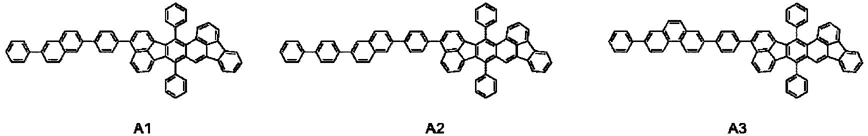

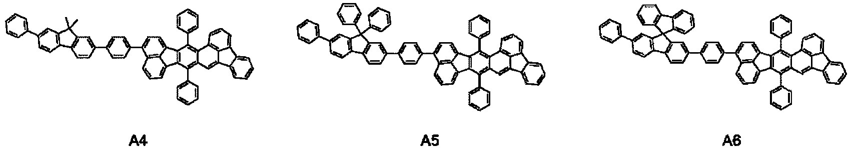

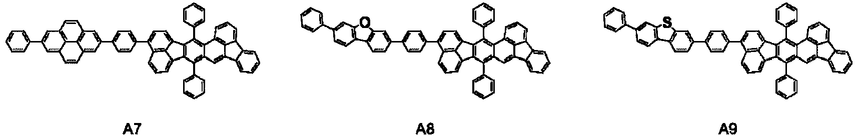

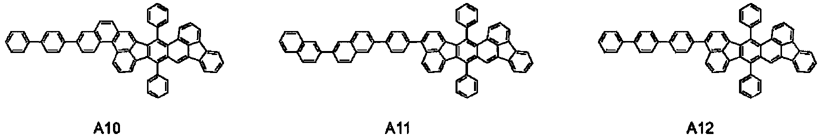

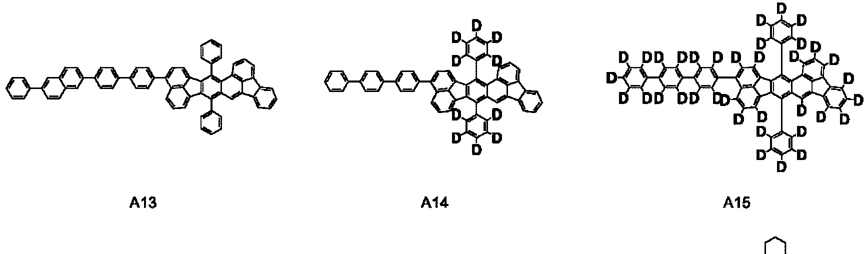

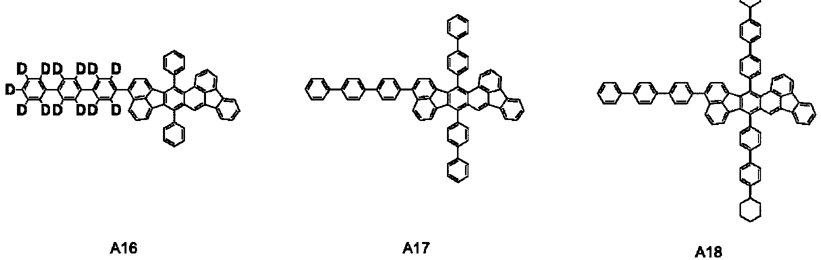

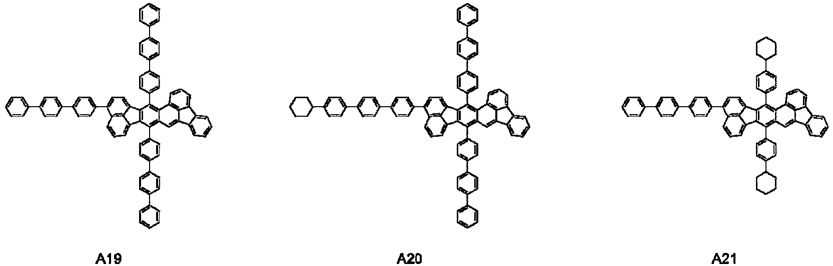

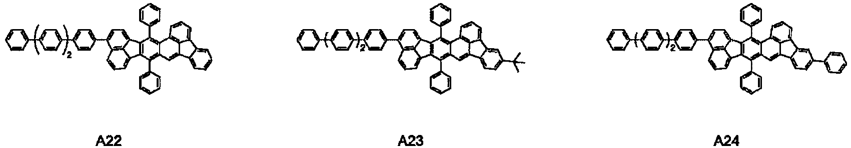

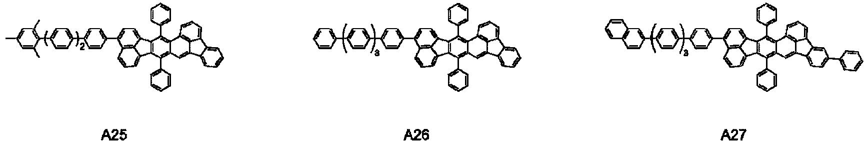

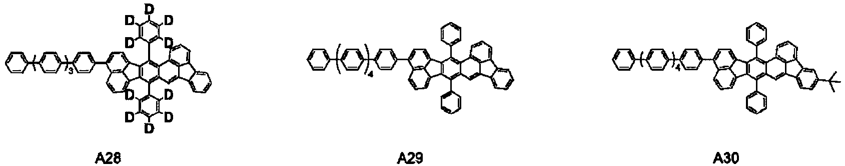

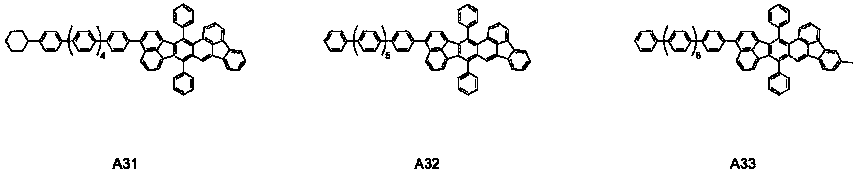

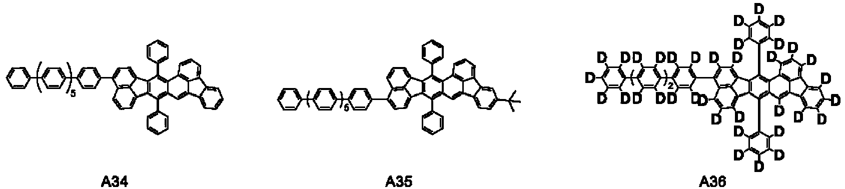

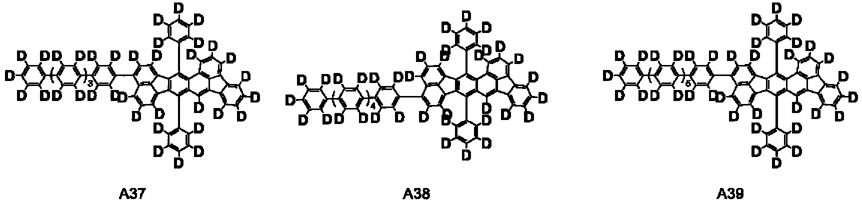

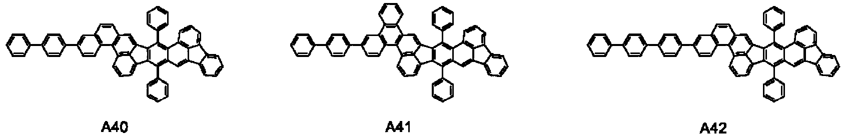

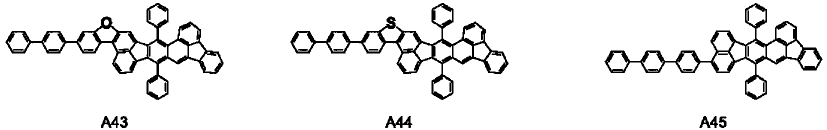

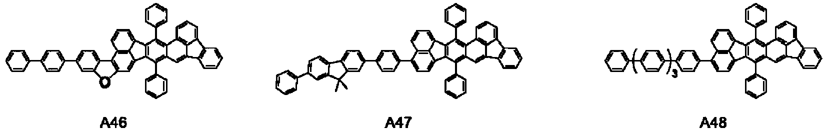

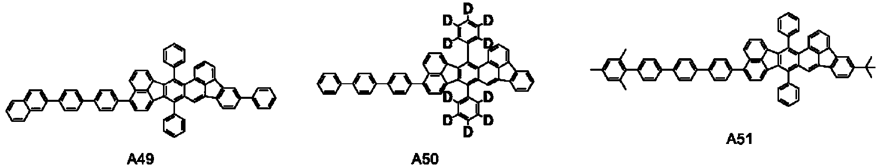

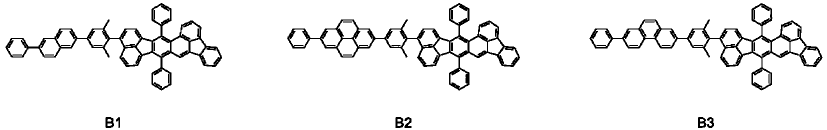

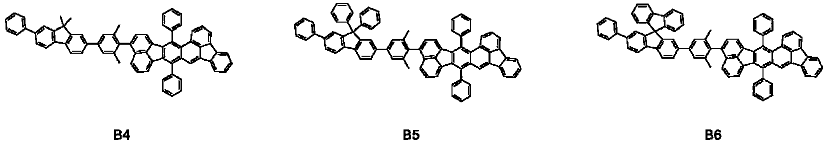

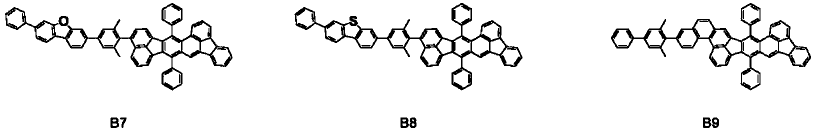

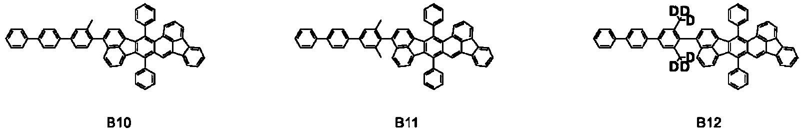

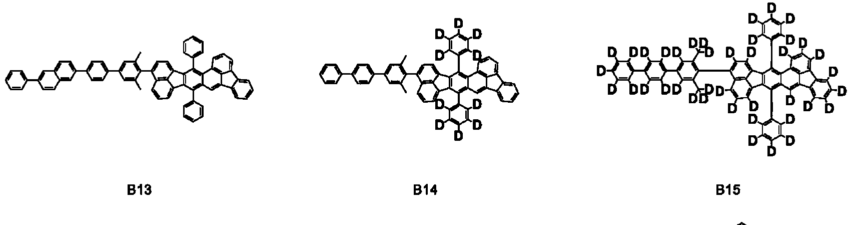

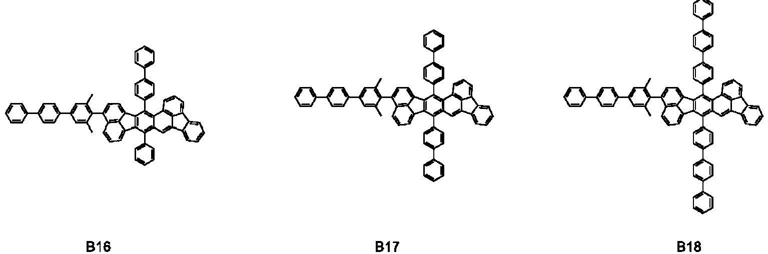

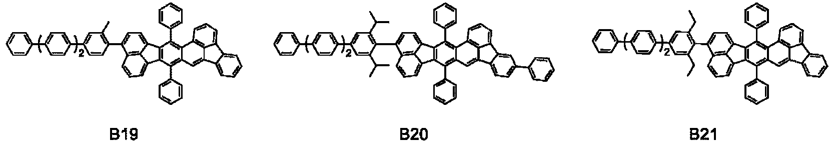

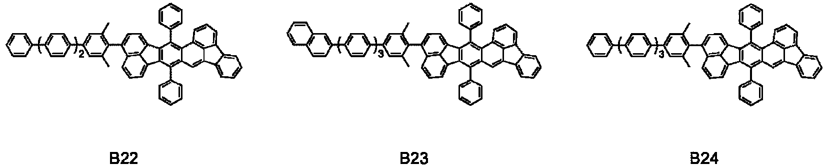

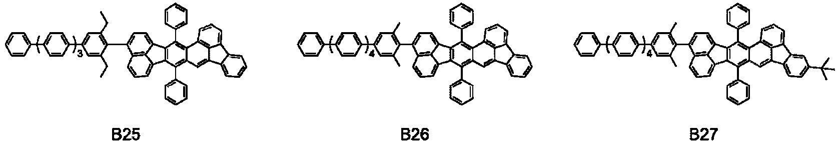

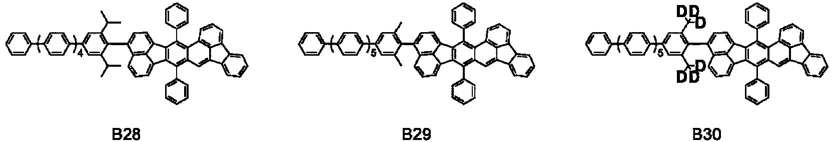

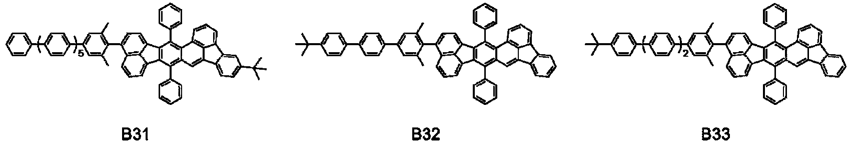

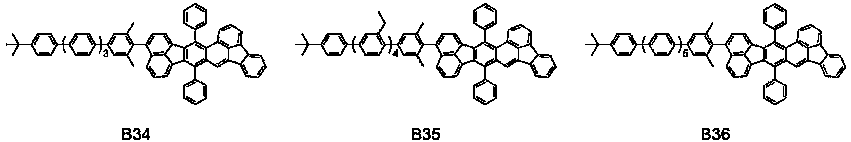

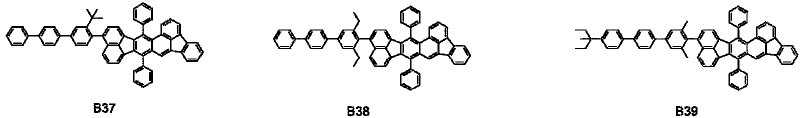

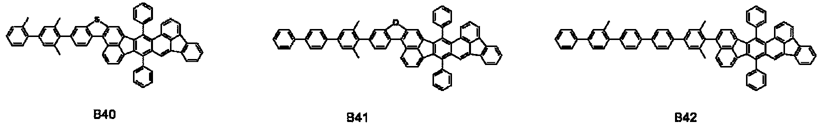

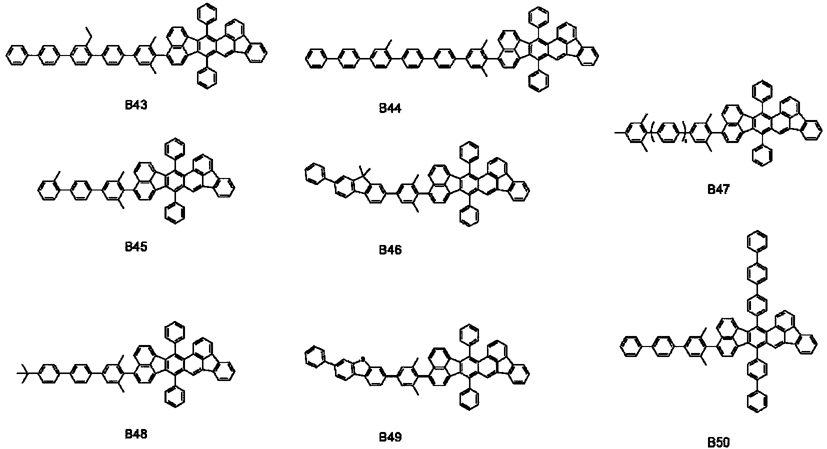

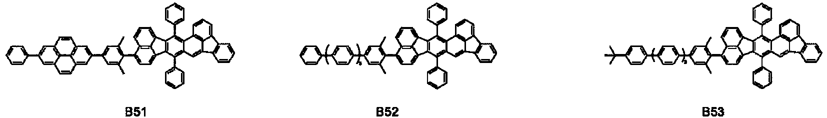

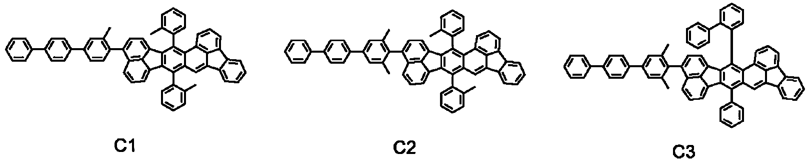

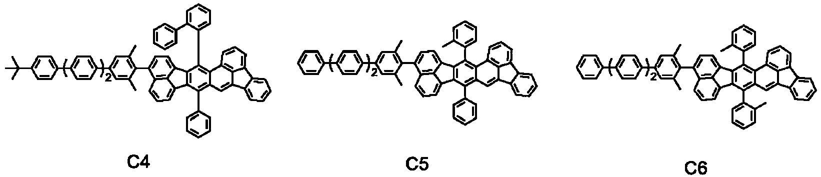

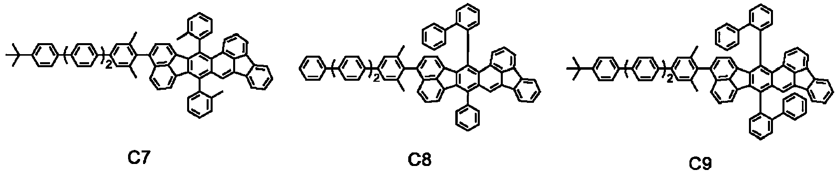

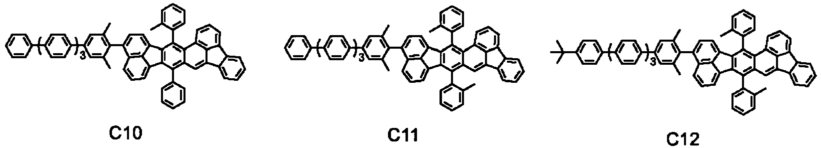

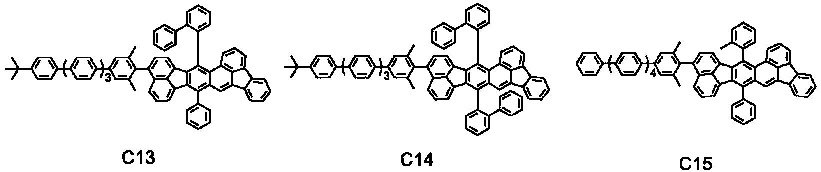

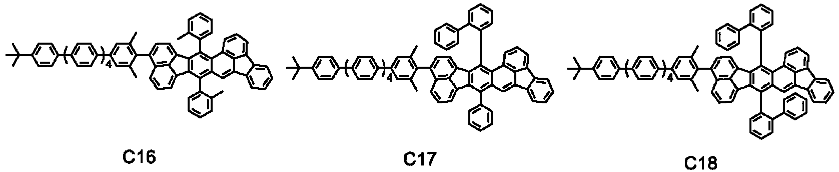

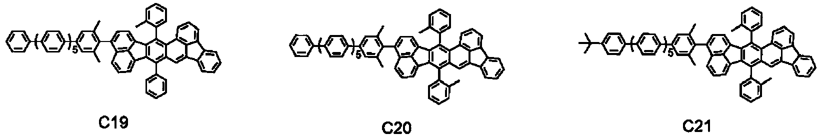

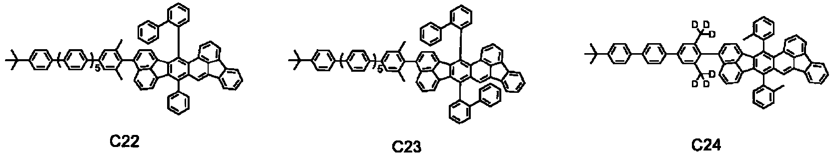

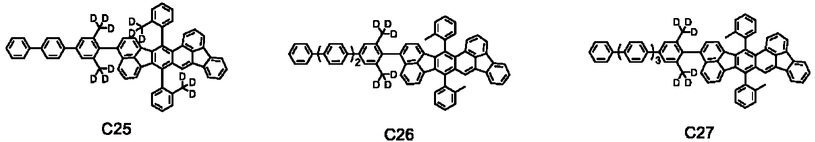

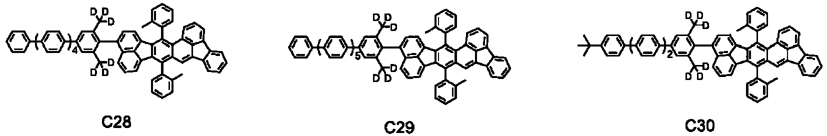

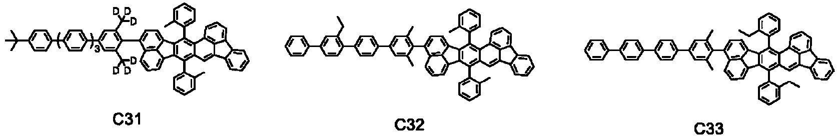

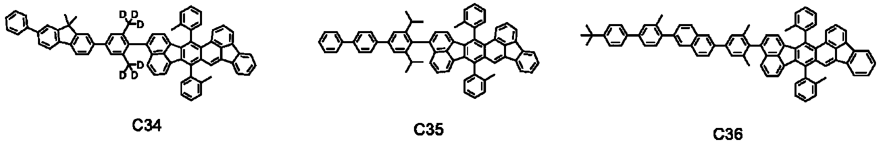

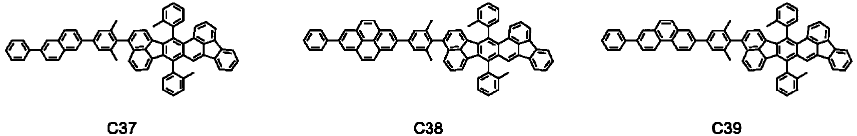

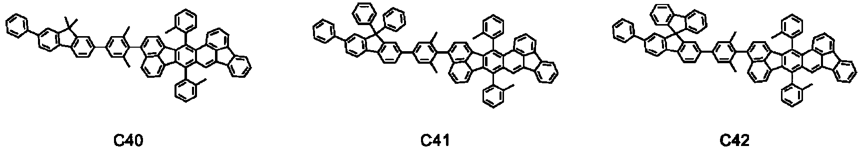

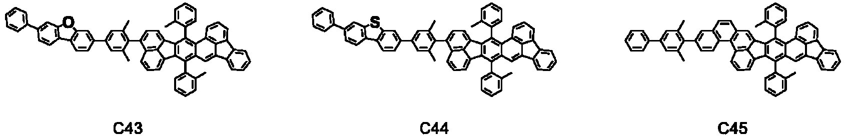

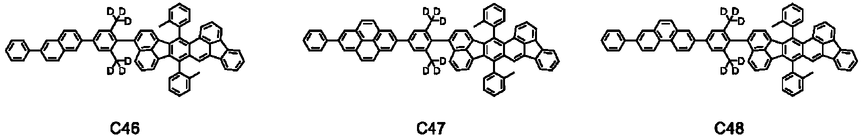

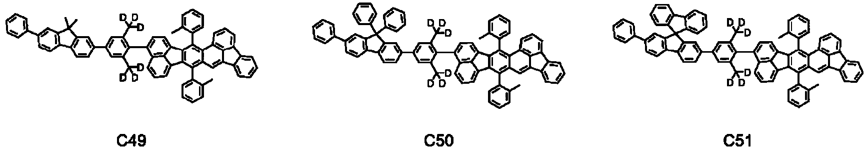

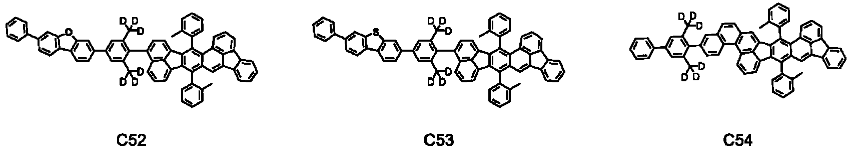

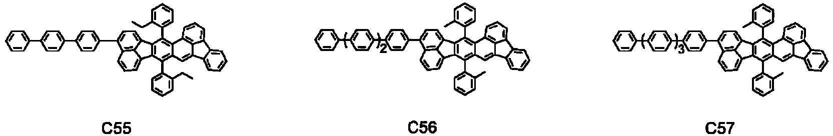

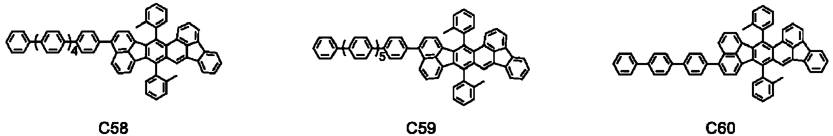

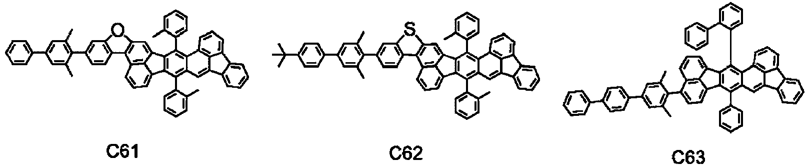

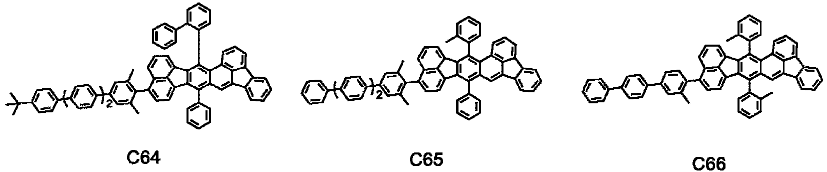

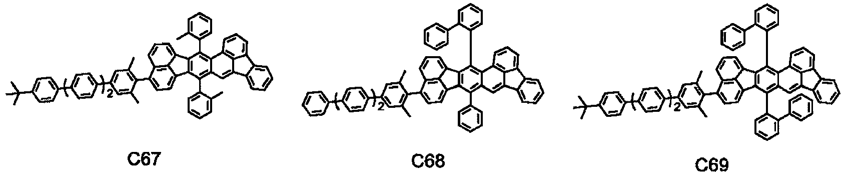

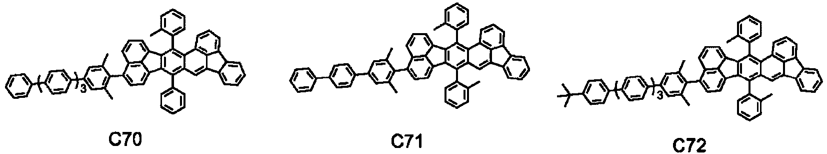

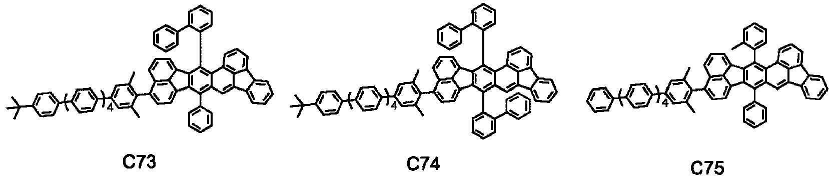

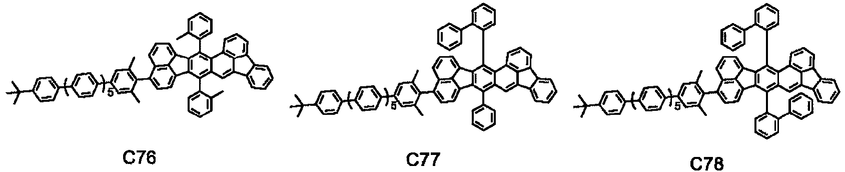

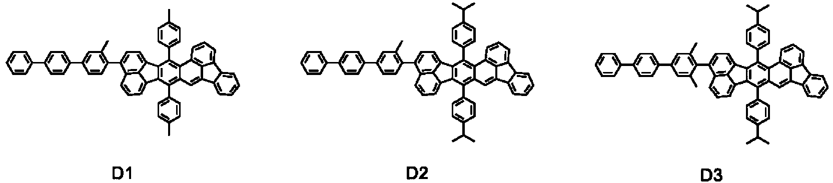

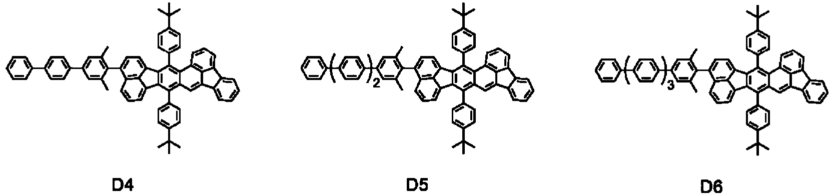

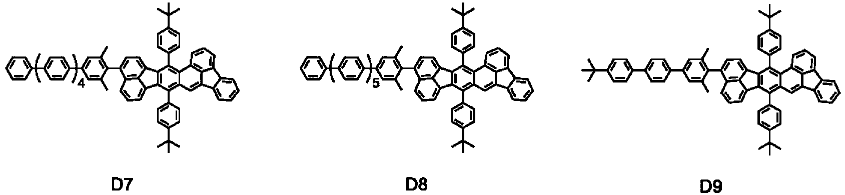

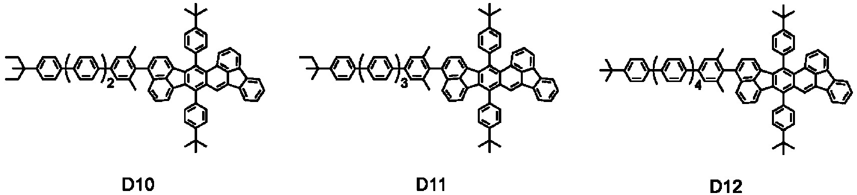

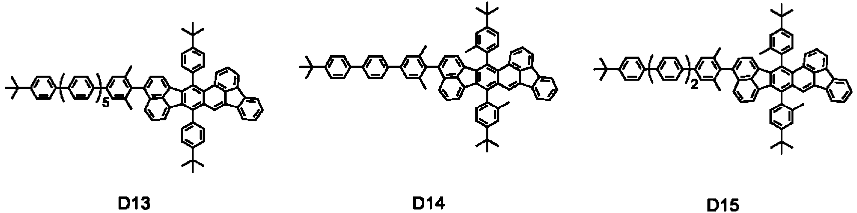

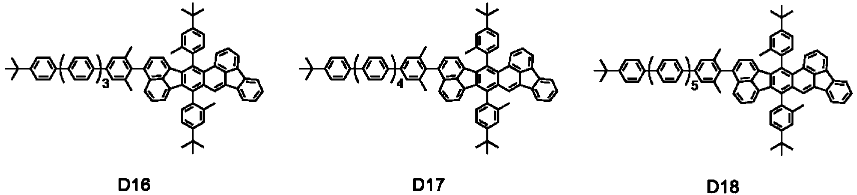

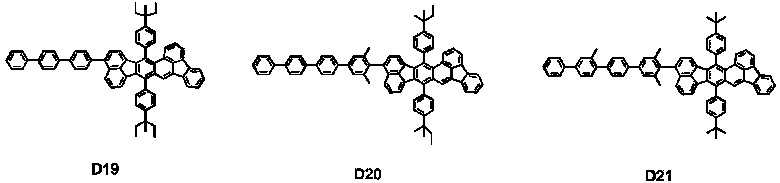

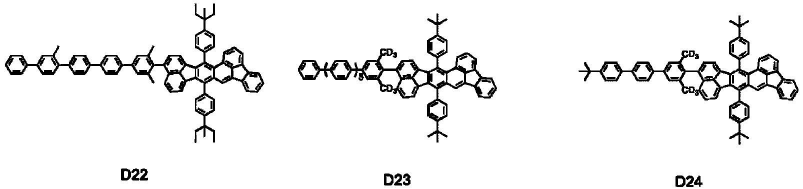

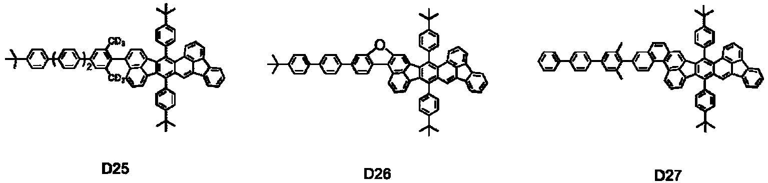

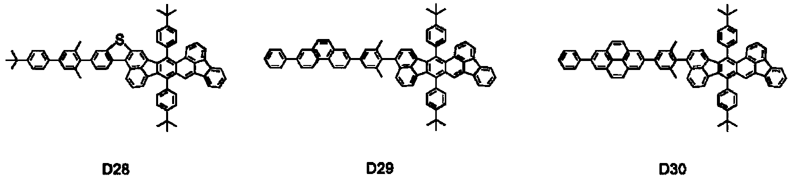

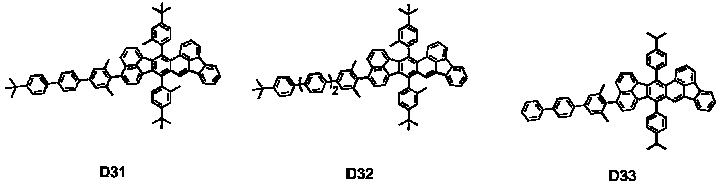

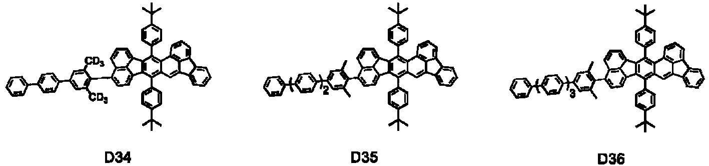

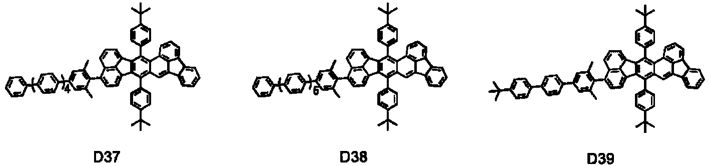

Among these exemplary compounds, the compound group of Group A includes compounds in which R₂₄ and R₂₇ are hydrogen atoms or deuterium atoms. Thus, the compounds belonging to Group A exhibit effects of emitting light with a longer wavelength among the organic compounds according to the present embodiment and having particularly high durability.

Among the above exemplary compounds, the compounds belonging to Group B are compounds in which at least one of R₂₄ and R₂₇ denotes an alkyl group. Among the phenyl groups directly bonded to the basic skeleton, a substituent at an ortho position with respect to the basic skeleton twists a phenyl group with respect to the basic skeleton and suppresses the extension of π conjugation. Thus, the compounds belonging to Group B exhibit effects of having a shorter emission wavelength and emitting blue light with high color purity.

Among the above exemplary compounds, in the compounds belonging to Group C, at least one of R₁, R₅, R₁₄, and R₁₈ in the general formula [1] has a substituent. Thus, the basic skeleton twisted with respect to the phenyl group bonded in the minor-axis direction of the basic skeleton reduces the intermolecular stacking and improves the sublimability. Thus, the compounds belonging to Group C have higher element durability.

Among the above exemplary compounds, the compounds belonging to Group D have a bulky substituent in any of R₃, R₁₆, and Ar in the general formula [1]. This reduces the intermolecular stacking, and therefore a compound belonging to Group D, when used as a guest material of a light-emitting layer, exhibits effects of suppressing the concentration quenching and improving the PLQY.

### (2) Organic Light-Emitting Element

Next, an organic light-emitting element according to an embodiment of the present embodiment will be described. An organic light-emitting element according to an embodiment of the present embodiment includes a first electrode, a second electrode, and an organic compound layer between the electrodes. One of the first electrode and the second electrode is a positive electrode, and the other is a negative electrode. In the organic light-emitting element according to the present embodiment, the organic compound layer may be a single layer or a laminate composed of a plurality of layers, provided that the organic compound layer includes a light-emitting layer. An organic compound according to the present embodiment may be contained in the organic compound layer and is preferably contained in the light-emitting layer. When the organic compound layer is a laminate composed of a plurality of layers, the organic compound layer may include, in addition to the light-emitting layer, a hole injection layer, a hole transport layer, an electron-blocking layer, a hole/exciton-blocking layer, an electron transport layer, an electron injection layer, and the like. The light-emitting layer may be a single layer or a laminate composed of a plurality of layers. When the light-emitting layer is composed of a plurality of layers, a charge generation layer may be provided between the layers. The charge generation layer may be constituted by a compound having a lowest unoccupied molecular orbital (LUMO) energy level lower than the LUMO energy level of the hole transport layer and may have a LUMO energy level lower than the HOMO energy level of the hole transport layer. Here, the HOMO energy level and the LUMO energy level of the organic compound layer may be the HOMO energy level and the LUMO energy level of an organic compound having the highest weight ratio in the organic compound layer.

Here, the HOMO energy level and the LUMO energy level are described as being "higher" as their values approach the vacuum level. The LUMO energy level of the charge generation layer being lower than the HOMO energy level of the hole transport layer means that the LUMO energy level of the charge generation layer is farther from the vacuum level than the HOMO energy level of the hole transport layer.

In the present specification, the HOMO energy level and the LUMO energy level can be calculated using molecular orbital calculation.

The HOMO energy level and the LUMO energy level in the present specification can also be calculated using an ionization potential and a band gap. The HOMO energy level can be estimated by measuring the ionization potential. The ionization potential can be measured with a measuring apparatus, such as AC-3, after dissolving a compound to be measured in a solvent, such as toluene, or forming a vapor-deposited film of a compound to be measured on a substrate, such as glass. The band gap can be measured by dissolving a compound to be measured in a solvent, such as toluene, followed by excitation light irradiation. The band gap can be determined by measuring an absorption edge of an absorption spectrum of the excitation light. Alternatively, the band gap can be determined by vapor-depositing a compound to be measured on a substrate, such as glass, and irradiating the vapor-deposited film with excitation light. The band gap can be determined by measuring an absorption edge of an absorption spectrum in which the vapor-deposited film absorbs excitation light.

The LUMO energy level can be calculated using the band gap and the value of the ionization potential. The LUMO energy level can be estimated by subtracting the value of the ionization potential from the band gap.

The LUMO energy level can also be estimated from the reduction potential. For example, one-electron reduction potential is estimated by cyclic voltammetry (CV) measurement. The CV can be measured, for example, in 0.1 M tetrabutylammonium perchlorate in DMF using Ag/Ag⁺ as a reference electrode, Pt as a counter electrode, and glassy carbon as a working electrode. The LUMO energy level can be estimated by adding the difference between the reduction potential of the compound and the reduction potential of ferrocene to -4.8 eV.

In an organic light-emitting element according to an embodiment of the present embodiment, when an organic compound according to the present embodiment is contained in a light-emitting layer, the light-emitting layer may be a layer composed only of the organic compound according to the present embodiment or may be a layer composed of the organic compound according to the present embodiment and another compound. When the light-emitting layer is a layer composed of the organic compound according to the present embodiment and another compound, the organic compound according to the present embodiment may be used as a host material or a guest material of the light-emitting layer. It may also be used as an assist material that can be contained in the light-emitting layer. The term "host material", as used herein, is also referred to as a "host" or a "first compound" and refers to a compound having the highest mass ratio among the compounds constituting the light-emitting layer. The term "guest material", as used herein, is also referred to as a "guest", a "dopant material", a "dopant", or a "third compound", and is a compound that has a lower mass ratio than that of the host among the compounds constituting the light-emitting layer and that is responsible for main light emission. Thus, the guest material is sometimes also referred to as a light-emitting material. The assist material is also referred to as an "assist" or a "second compound" and is a compound that has a lower mass ratio than the host material among the compounds constituting the light-emitting layer and that assists light emission of the guest material. The assist material is also referred to as a second host.

Here, the lowest singlet excitation energy of the host material is denoted by S1(H), the lowest singlet excitation energy of the guest material is denoted by S1(D), and the lowest singlet excitation energy of the assist material is denoted by S1(A). The guest material may be regarded as an organic compound according to the present embodiment. At this time, the organic light-emitting element according to the present embodiment preferably satisfies S1(H) > S1(D) or S1(H) > S1(A) > S1(D). When the lowest singlet excitation energy of a compound contained in the organic light-emitting element according to the present embodiment satisfies the above relationship, an exciton can be efficiently transferred to the guest material, and therefore the organic light-emitting element has higher light emission efficiency.

When an organic compound according to the present embodiment is used as the guest material of the light-emitting layer, the concentration of the guest material may be 0.01% or more by mass and less than 50% by mass, preferably 0.01% or more by mass and 20% or less by mass, more preferably 0.01% or more by mass and 10% or less by mass, still more preferably 0.01% or more by mass and 5% or less by mass, relative to the entire light-emitting layer.

When the light-emitting layer further contains an assist material, the assist material may constitute 1% or more by mass and less than 50% by mass, preferably 10% or more by mass and less than 50% by mass, relative to the entire light-emitting layer. The guest may constitute 0.01% or more by mass and 20% or less by mass, preferably 0.01% or more by mass and 5% or less by mass.

The present inventors have conducted various studies and have found that an organic compound according to the present embodiment can be used as a host material or a guest material in the light-emitting layer, particularly as a guest material in the light-emitting layer, to provide an element that outputs bright light with high efficiency and has very high durability. The light-emitting layer may be of monolayer or multilayer or may contain a light-emitting material with another emission color to mix the emission color with the blue emission color of the present embodiment. The term "multilayer", as used herein, refers to a laminate of a light-emitting layer and another light-emitting layer. In this case, the emission color of the organic light-emitting element is not limited to blue. More specifically, the color may be white or a neutral color. For white color emission, another light-emitting layer emits light of a color other than blue, such as red or green. The film is formed by vapor deposition or coating. This will be described in detail in exemplary embodiments below.

An organic compound according to the present embodiment can be used as a constituent material for an organic compound layer other than the light-emitting layer constituting the organic light-emitting element according to the present embodiment. More specifically, the organic compound may be used as a constituent material of an electron transport layer, an electron injection layer, a hole transport layer, a hole injection layer, a hole-blocking layer, or the like. In such a case, the emission color of the organic light-emitting element is not limited to blue. More specifically, the emission color may be white or a neutral color.

### (3) Other Compounds

Here, in addition to an organic compound according to the present embodiment, if necessary, a known low-molecular-weight or high-molecular-weight hole injection compound or hole transport compound, host material, light-emitting compound, electron injection compound, electron transport compound, or the like can be used together. Examples of these compounds are described below.

The hole injection/transport material is preferably a material that facilitates hole injection from the positive electrode and that has high hole mobility to transport an injected hole to a light-emitting layer. Furthermore, to reduce degradation of film quality, such as crystallization, in the organic light-emitting element, a material with a high glass transition temperature is preferred. A low-molecular-weight or high-molecular-weight material with hole injection/transport ability may be a triarylamine derivative, an aryl carbazole derivative, a phenylenediamine derivative, a stilbene derivative, a phthalocyanine derivative, a porphyrin derivative, polyvinylcarbazole, polythiophene, or another electrically conductive polymer. Furthermore, the hole injection/transport material is also suitable for use in an electron-blocking layer. Specific examples of a compound that can be used as a hole injection/transport material include, but are not limited to, the following.

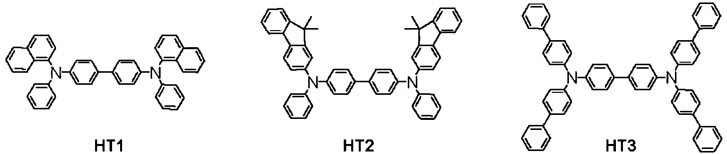

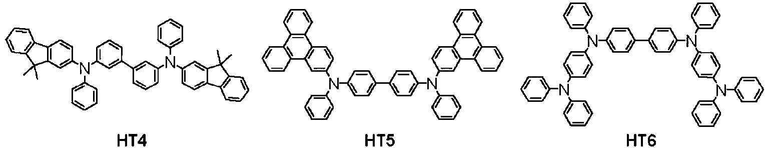

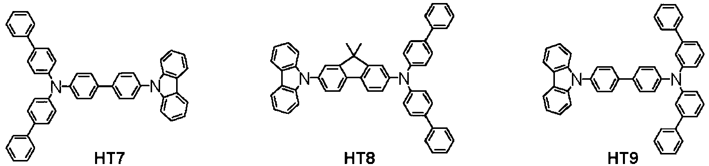

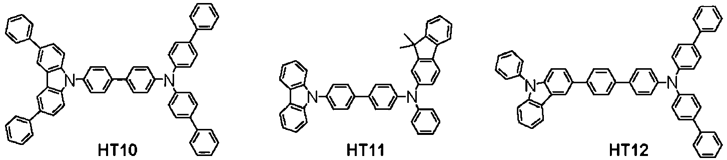

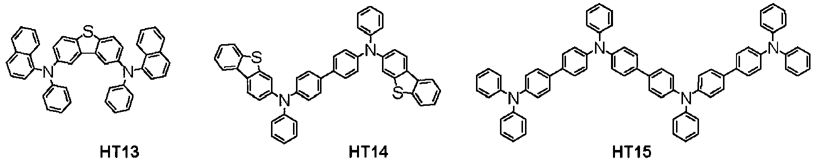

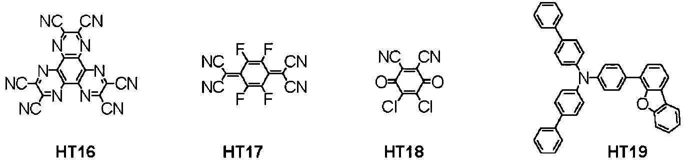

A guest material mainly related to the light-emitting function may be, in addition to an organic compound represented by the general formula [1], a fused-ring compound (for example, a fluorene derivative, a naphthalene derivative, a pyrene derivative, a perylene derivative, a tetracene derivative, an anthracene derivative, rubrene, or the like), a quinacridone derivative, a coumarin derivative, a stilbene derivative, an organoaluminum complex, such as tris(8-quinolinolato)aluminum, an iridium complex, a platinum complex, a rhenium complex, a copper complex, a europium complex, a ruthenium complex, or a polymer derivative, such as a poly(phenylene vinylene) derivative, a polyfluorene derivative, or a polyphenylene derivative. Specific examples of a compound that can be used as a light-emitting material include, but are not limited to, the following.

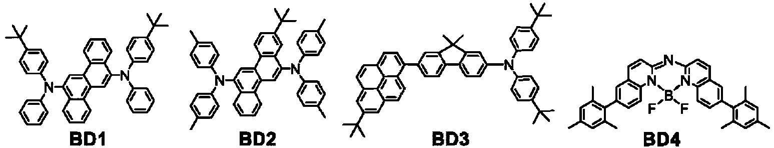

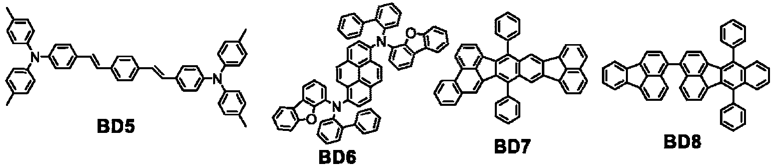

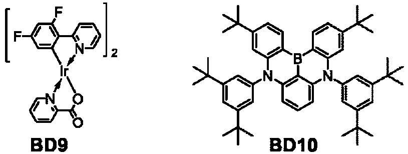

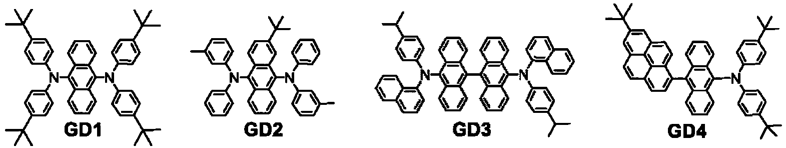

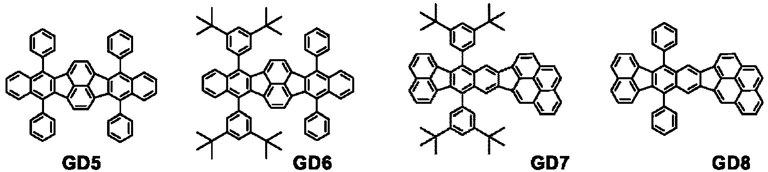

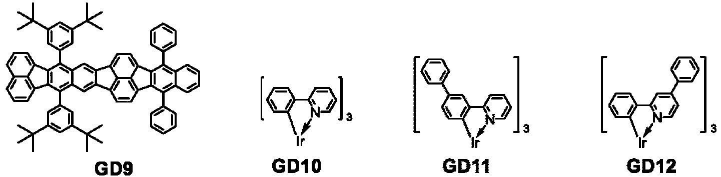

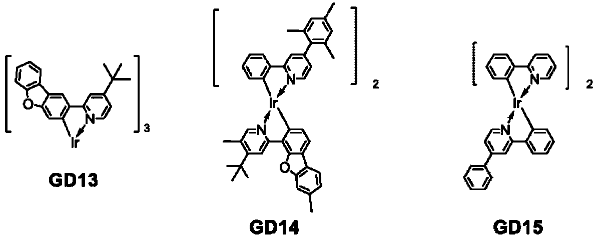

A host material or an assist material in a light-emitting layer may be an aromatic hydrocarbon compound or a derivative thereof, a carbazole derivative, a dibenzofuran derivative, a dibenzothiophene derivative, an organoaluminum complex, such as tris(8-quinolinolato)aluminum, an organoberyllium complex, or the like. Specific examples of a compound used as a light-emitting layer host or a light-emitting assist material in a light-emitting layer include, but are not limited to, the following.

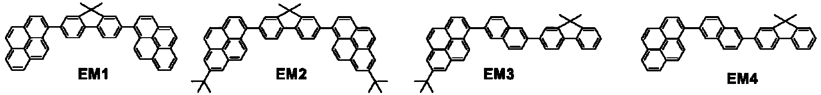

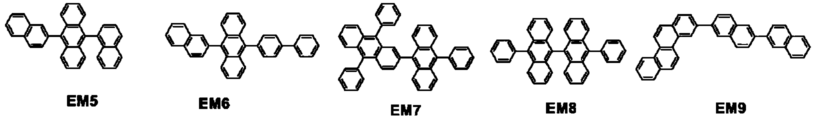

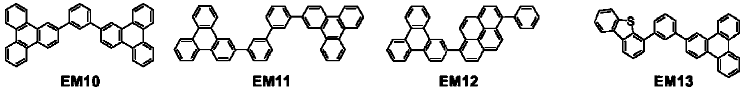

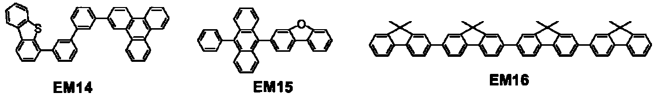

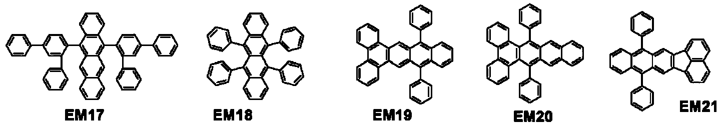

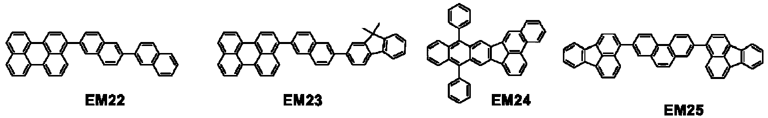

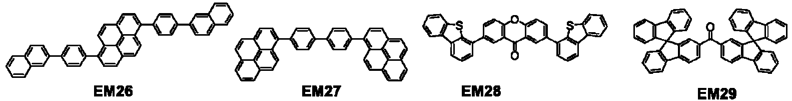

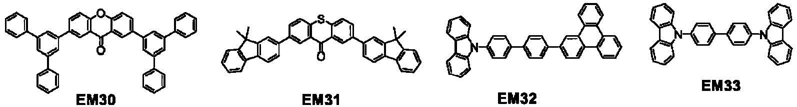

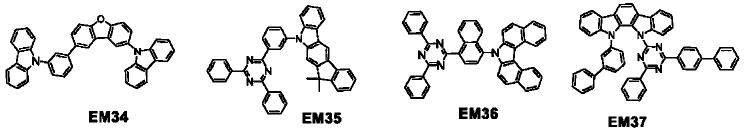

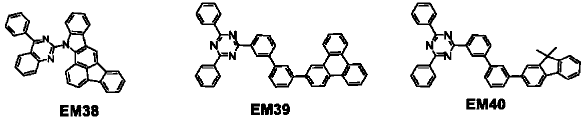

Among EM1 to EM40, the host material may be a hydrocarbon compound having a fused polycyclic hydrocarbon group. Specific examples include EM1 to EM12 and EM16 to EM27.

The electron transport material can be arbitrarily selected from those capable of transporting an electron injected from a negative electrode to a light-emitting layer and is selected in consideration of the balance with the hole mobility of the hole transport material, or the like. A material with electron transport ability may be an oxadiazole derivative, an oxazole derivative, a pyrazine derivative, a triazole derivative, a triazine derivative, a quinoline derivative, a quinoxaline derivative, a phenanthroline derivative, an organoaluminum complex, a fused-ring compound (for example, a fluorene derivative, a naphthalene derivative, a chrysene derivative, an anthracene derivative, or the like), or the like. Furthermore, the electron transport material is also suitable for use in a hole-blocking layer. Specific examples of a compound that can be used as an electron transport material include, but are not limited to, the following.

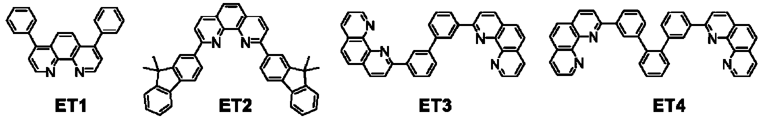

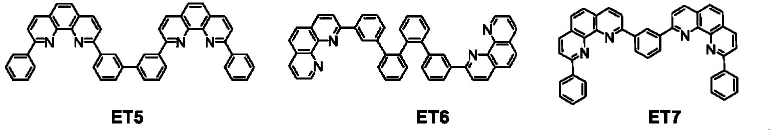

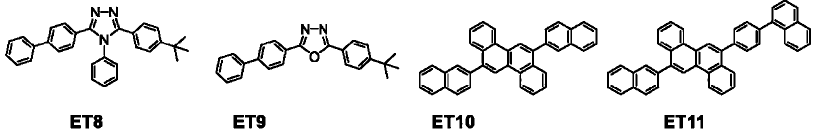

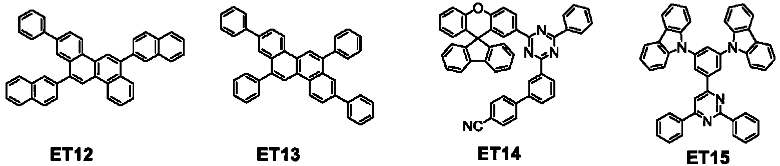

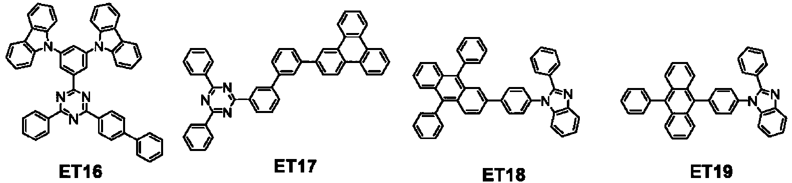

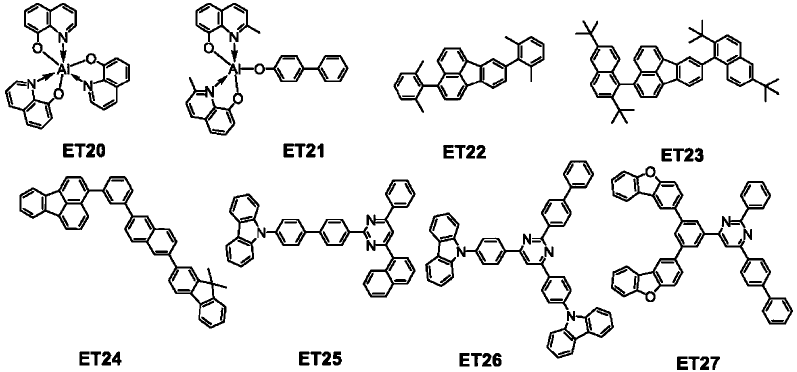

The electron injection material can be arbitrarily selected from materials that can easily inject an electron from a negative electrode and is selected in consideration of the balance with the hole injection properties, or the like. An n-type dopant or a reducing dopant may be contained as an organic compound. Examples thereof include a compound containing an alkali metal, such as lithium fluoride, a lithium complex, such as lithium quinolinol, a benzimidazolidene derivative, an imidazolidene derivative, a fulvalene derivative, and an acridine derivative.

### (4) Configuration of Organic Light-emitting Element

Constituent members constituting the organic light-emitting element according to the present embodiment will be described below.

The organic light-emitting element includes an insulating layer, a first electrode, an organic compound layer, and a second electrode on a substrate. A protective layer, a color filter, a microlens, or the like may be provided on the second electrode. When a color filter is provided, a planarization layer may be provided between a protective layer and the color filter. The planarization layer can be composed of an acrylic resin or the like. The same applies to the case where a planarization layer is provided between a color filter and a microlens.

### [Substrate]

The substrate may be quartz, glass, a silicon wafer, a resin, a metal, or the like. Furthermore, a switching element, such as a transistor, or a wire may be provided on the substrate, and an insulating layer may be provided thereon. The insulating layer may be composed of any material, provided that the insulating layer can have a contact hole for wiring between the insulating layer and the first electrode and is insulated from unconnected wires. For example, the insulating layer may be formed of a resin, such as polyimide, silicon oxide, or silicon nitride.

### [Electrode]

A pair of electrodes can be used as the electrodes. The pair of electrodes is a first electrode and a second electrode. More specifically, the pair of electrodes may be a positive electrode and a negative electrode. When an electric field is applied in a direction in which the organic light-emitting element emits light, the electrode with a higher potential is a positive electrode and the other is a negative electrode. It can also be said that the electrode that supplies holes to a light-emitting layer is a positive electrode and the electrode that supplies electrons is a negative electrode.

A constituent material of the positive electrode can have as large a work function as possible. Examples thereof include a metal element, such as gold, platinum, silver, copper, nickel, palladium, cobalt, selenium, vanadium, or tungsten, a mixture thereof, an alloy thereof, and a metal oxide, such as tin oxide, zinc oxide, indium oxide, indium tin oxide (ITO), or indium zinc oxide. An electrically conductive polymer, such as polyaniline, polypyrrole, or polythiophene, may also be used.

These electrode materials may be used alone or in combination. The positive electrode may be composed of one layer or a plurality of layers.

In the case of being used as a reflective electrode, for example, chromium, aluminum, silver, titanium, tungsten, molybdenum, an alloy thereof, a laminate thereof, or the like can be used. These materials can also function as a reflective film that does not have a role as an electrode. In the case of being used as a transparent electrode, an oxide transparent conductive layer, such as indium tin oxide (ITO) or indium zinc oxide, can be used, but the electrode is not limited thereto. The electrodes may be formed by photolithography.

A constituent material of the negative electrode can be a material with a small work function. For example, an alkali metal, such as lithium, an alkaline-earth metal, such as calcium, a metal element, such as aluminum, titanium, manganese, silver, lead, or chromium, or a mixture thereof may be used. An alloy of these metal elements may also be used. For example, magnesium-silver, aluminum-lithium, aluminum-magnesium, silver-copper, or zincsilver may be used. A metal oxide, such as indium tin oxide (ITO), can also be used. These electrode materials may be used alone or in combination. The negative electrode may be composed of one layer or a plurality of layers. Among these, silver is preferably used, and a silver alloy is more preferably used to reduce aggregation of silver. Provided that the aggregation of silver can be reduced, the ratio of the alloy is not particularly limited. For example, the ratio of silver to another metal may be 1:1, 3:1, or the like.

The negative electrode may be, but is not limited to, an oxide electroconductive layer, such as ITO, for a top emission element or a reflective electrode, such as aluminum (Al), for a bottom emission element. The method for forming the negative electrode is not particularly limited, but it is more preferable to use a DC or AC sputtering method or the like because the coverage of the film is good and the resistance is easily lowered.

### [Organic Compound Layer]

The organic compound layer may be formed of a single layer or a plurality of layers. Depending on their functions, the plurality of layers may be referred to as a hole injection layer, a hole transport layer, an electron-blocking layer, a light-emitting layer, a hole-blocking layer, an electron transport layer, or an electron injection layer. The organic compound layer is composed mainly of an organic compound and may contain an inorganic atom or an inorganic compound. For example, copper, lithium, magnesium, aluminum, iridium, platinum, molybdenum, zinc, or the like may be contained. The organic compound layer may be disposed between the first electrode and the second electrode and may be disposed in contact with the first electrode and the second electrode.

The organic compound layer (a hole injection layer, a hole transport layer, an electron-blocking layer, a light-emitting layer, a hole-blocking layer, an electron transport layer, an electron injection layer, or the like) constituting the organic light-emitting element according to the present embodiment is formed by the method described below.

The organic compound layer constituting the organic light-emitting element according to the present embodiment can be formed by a dry process, such as a vacuum deposition method, an ionized deposition method, sputtering, or plasma. Instead of the dry process, a wet process may also be employed in which a layer is formed by a known coating method (for example, spin coating, dipping, a casting method, an LB method, an ink jet method, or the like) using an appropriate solvent.

Here, when a layer is formed by a vacuum deposition method, a solution coating method, or the like, crystallization or the like is less likely to occur, which results in high temporal stability. When a film is formed by a coating method, the film can be formed in combination with an appropriate binder resin.

The binder resin may be, but is not limited to, a polyvinylcarbazole resin, a polycarbonate resin, a polyester resin, an ABS resin, an acrylic resin, a polyimide resin, a phenolic resin, an epoxy resin, a silicone resin, a urea resin, or the like.

These binder resins may be used alone as a homopolymer or a copolymer or may be used as a mixture of two or more thereof. Furthermore, if necessary, a known additive agent, such as a plasticizer, an oxidation inhibitor, or an ultraviolet absorbent, may be used in combination.

### [Protective Layer]

A protective layer may be provided on the negative electrode. For example, a glass sheet with a moisture absorbent may be attached to the negative electrode to reduce the amount of water or the like entering the organic compound layer and to reduce the occurrence of display defects. In another embodiment, a passivation film of silicon nitride or the like may be provided on the negative electrode to reduce the amount of water or the like entering the organic compound layer. For example, after the negative electrode is formed, the negative electrode is transferred to another chamber without breaking the vacuum, and a silicon nitride film with a thickness of 2 µm may be formed as a protective layer by a chemical vapor deposition (CVD) method. After the film formation by the CVD method, a protective layer may be formed by an atomic layer deposition method (ALD method). A film formed by the ALD method may be formed of any material, such as silicon nitride, silicon oxide, or aluminum oxide. Silicon nitride may be further formed by the CVD method on the film formed by the ALD method. The film formed by the ALD method may have a smaller thickness than the film formed by the CVD method. More specifically, the film thickness of the film formed by the ALD method may be 50% or less or even 10% or less of the film thickness of the film formed by the CVD method.

### [Color Filter]

A color filter may be provided on the protective layer. For example, a color filter may be provided on another substrate in consideration of the size of the organic light-emitting element, and the other substrate may be bonded to the substrate on which the organic light-emitting element is provided, or the color filter may be patterned over the above-described protective layer by a photolithography technique. The color filter may be composed of a polymer.

### [Planarization Layer]

A planarization layer may be provided between the color filter and the protective layer. The planarization layer is provided to reduce the roughness of the underlayer. The planarization layer is sometimes referred to as a material resin layer with any purpose. The planarization layer may be composed of an organic compound, which may have a low molecular weight or a high molecular weight, preferably a high molecular weight.

The planarization layer may be provided above and below the color filter, and the constituent materials thereof may be the same or different. Specific examples thereof include a polyvinylcarbazole resin, a polycarbonate resin, a polyester resin, an ABS resin, an acrylic resin, a polyimide resin, a phenolic resin, an epoxy resin, a silicone resin, and a urea resin.

### [Microlens]

The organic light-emitting element according to the present embodiment may have an optical member, such as a microlens, on the light emission side. The microlens may be composed of an acrylic resin, an epoxy resin, or the like. The microlens may be used to increase the amount of light extracted from the organic light-emitting element and control the direction of the extracted light. The microlens may have a hemispherical shape. For a hemispherical microlens, the vertex of the microlens is a contact point between the hemisphere and a tangent line parallel to the insulating layer among the tangent lines in contact with the hemisphere. The vertex of the microlens in any cross-sectional view can be determined in the same manner. More specifically, the vertex of the microlens in a cross-sectional view is a contact point between the semicircle of the microlens and a tangent line parallel to the insulating layer among the tangent lines in contact with the semicircle.

It is also possible to define the midpoint of the microlens. In a cross section of the microlens, a midpoint of a line segment from one end point to the other end point of the arc can be referred to as a midpoint of the microlens. A cross section in which the vertex and the midpoint are determined may be a cross section perpendicular to the insulating layer.

### [Opposite Substrate]

An opposite substrate may be provided on the planarization layer. The opposite substrate is provided at a position corresponding to the above-described substrate and is therefore referred to as an opposite substrate. The constituent material of the opposite substrate may be the same as that of the above-described substrate. When the above-described substrate is a first substrate, the opposite substrate may be a second substrate.

### [Pixel Circuit]

A light-emitting apparatus may include a pixel circuit coupled to the light-emitting element. The pixel circuit may be of an active matrix type, which independently controls the light emission of a first light-emitting element and a second light-emitting element. The active matrix type circuit may be voltage-programmed or current-programmed. The drive circuit has a pixel circuit for each pixel. The pixel circuit may include a light-emitting element, a transistor for controlling the luminous brightness of the light-emitting element, a transistor for controlling light emission timing, a capacitor for holding the gate voltage of the transistor for controlling the luminous brightness, and a transistor for GND connection without through the light-emitting element.

A light-emitting apparatus includes a display region and a peripheral region around the display region. The display region includes the pixel circuit, and the peripheral region includes a display control circuit. The mobility of a transistor constituting the pixel circuit may be smaller than the mobility of a transistor constituting the display control circuit.

The gradient of the current-voltage characteristics of a transistor constituting the pixel circuit may be smaller than the gradient of the current-voltage characteristics of a transistor constituting the display control circuit. The gradient of the current-voltage characteristics can be determined by so-called Vg-Ig characteristics.

A transistor constituting the pixel circuit is a transistor coupled to a light-emitting element, such as a first light-emitting element.

### [Pixel]

The organic light-emitting element has a plurality of pixels. Each pixel includes a sub-pixel that emits light of a different color. The sub-pixels may have, for example, RGB emission colors.

In each pixel, a region also referred to as a pixel aperture emits light. This region is the same as the first region. The pixel aperture may be 15 µm or less or 5 µm or more. More specifically, the pixel aperture may be 11 µm, 9.5 µm, 7.4 µm, 6.4 µm, or the like.

The distance between the sub-pixels may be 10 µm or less, more specifically, 8 µm, 7.4 µm, or 6.4 µm.

The pixels may be arranged in a known form in a plan view. Examples thereof include a stripe arrangement, a delta arrangement, a PenTile arrangement, and a Bayer arrangement. Each sub-pixel may have any known shape in a plan view. Examples thereof include quadrangles, such as a rectangle and a rhombus, and a hexagon. As a matter of course, the rectangle also includes a figure that is not strictly rectangular but is close to rectangular. The shape of each sub-pixel and the pixel array can be used in combination.

(5) Applications of Organic Light-emitting Element According to Present Embodiment
The organic light-emitting element according to the present embodiment can be used as a constituent member of an image display apparatus, a display apparatus, a lighting apparatus, or the like. Other applications include a display unit of an image display apparatus including the display unit and a housing in which the display unit is provided, an exposure light source of an electrophotographic image-forming apparatus, a backlight of a liquid crystal display apparatus, and a light-emitting apparatus including a color filter in a white light source.

The display apparatus may be an image information processor that includes an image input unit that inputs image information from an area CCD, a linear CCD, a memory card, or the like, includes an information processing unit that processes the input information, and displays the input image on a display unit.

A display unit of an imaging apparatus or an ink jet printer may have a touch panel function. A method of driving the touch panel function may be, but is not limited to, an infrared method, a capacitance method, a resistive film method, or an electromagnetic induction method. Furthermore, the display apparatus may be used as a display unit of a multifunction printer.

Next, the display apparatus according to the present embodiment will be described below with reference to the accompanying drawings.

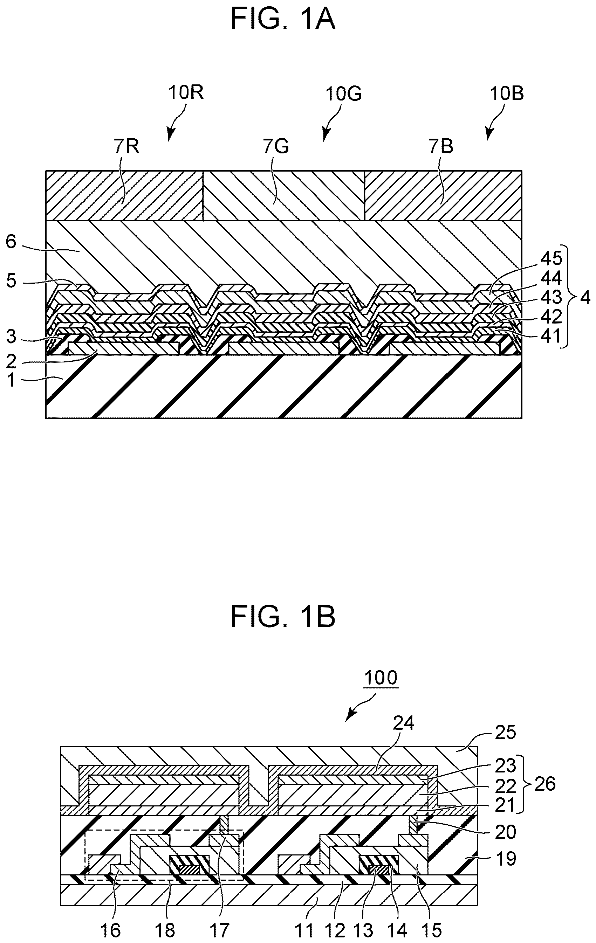

Figs. 1A and 1B are schematic cross-sectional views of an example of a display apparatus including an organic light-emitting element and a transistor coupled to the organic light-emitting element. The transistor is an example of an active element. The transistor may be a thin-film transistor (TFT).

Fig. 1A is an example of a pixel, which is a component of the display apparatus according to the present embodiment. The pixel includes sub-pixels 10. The sub-pixels are divided into 10R, 10G, and 10B according to their emission colors. The emission color may be distinguished by the wavelength of light emitted from the light-emitting layer, or light emitted from the sub-pixel may be selectively transmitted or color-converted by a color filter or the like. Each sub-pixel includes, on an interlayer insulating layer 1, a reflective electrode 2 serving as a first electrode, an insulating layer 3 covering an end of the reflective electrode 2, an organic compound layer 4 covering the first electrode and the insulating layer, a transparent electrode 5, a protective layer 6, and a color filter 7.

A transistor and/or a capacitor element may be provided under or inside the interlayer insulating layer 1. The transistor may be electrically connected to the first electrode via a contact hole (not shown) or the like.

The insulating layer 3 is also referred to as a bank or a pixel separation film. The insulating layer 3 covers the ends of the first electrode and surrounds the first electrode. A portion of the first electrode not covered with the insulating layer is in contact with the organic compound layer 4 and serves as a light-emitting region.

The organic compound layer 4 includes a hole injection layer 41, a hole transport layer 42, a first light-emitting layer 43, a second light-emitting layer 44, and an electron transport layer 45.

The second electrode 5 may be a transparent electrode, a reflective electrode, or a semitransparent electrode.

The protective layer 6 reduces the penetration of moisture into the organic compound layer. The protective layer is illustrated as a single layer but may be a plurality of layers. The protective layer 6 may include an inorganic compound layer and an organic compound layer.

The color filter 7 is divided into 7R, 7G, and 7B according to their colors. The color filter may be formed on a planarizing film (not shown). Furthermore, a resin protective layer (not shown) may be provided on the color filter. The color filter may be formed on the protective layer 6. Alternatively, the color filter 7 may be bonded after being provided on an opposite substrate, such as a glass substrate.

A display apparatus 100 in Fig. 1B includes an organic light-emitting element 26 and a TFT 18 as an example of a transistor. The display apparatus 100 includes a substrate 11 made of glass, silicon, or the like and an insulating layer 12 on the substrate 11. The display apparatus 100 includes, on the insulating layer, an active element 18, such as a TFT, and a gate electrode 13, a gate-insulating film 14, and a semiconductor layer 15 of the active element. The active element 18 also includes the semiconductor layer 15, the drain electrode 16, and the source electrode 17. An insulating film 19 is provided over the active element 18. A positive electrode 21 of the organic light-emitting element 26 is coupled to the source electrode 17 through a contact hole 20 formed in the insulating film.

The method for electrically connecting the electrodes (the positive electrode and a negative electrode) of the organic light-emitting element 26 to the electrodes (the source electrode and the drain electrode) of the TFT is not limited to the embodiment illustrated in Fig. 1B. More specifically, it is only necessary to electrically connect one of the positive electrode and the negative electrode to one of the source electrode and the drain electrode of the TFT. TFT refers to a thin-film transistor.

An organic compound layer 22 in the display apparatus 100 in Fig. 1B is illustrated as a single layer but may be composed of a plurality of layers. A first protective layer 24 and a second protective layer 25 for reducing deterioration of the organic light-emitting element are provided on a negative electrode 23.

The display apparatus 100 in Fig. 1B includes a transistor as a switching element but may include another switching element instead thereof.

The transistor used in the display apparatus 100 in Fig. 1B is not limited to a transistor including a single crystal silicon wafer and may also be a thin-film transistor including an active layer on an insulating surface of a substrate. The active layer may be single-crystal silicon, non-single-crystal silicon, such as amorphous silicon or microcrystalline silicon, or a non-single-crystal oxide semiconductor, such as indium zinc oxide or indium gallium zinc oxide. The thin-film transistor is also referred to as a TFT element.

The transistor in the display apparatus 100 in Fig. 1B may be formed within a substrate, such as a Si substrate. The phrase "formed within a substrate", as used herein, means that a transistor is produced by processing a substrate, such as a Si substrate, itself. In other words, when a transistor is included in a substrate, the substrate and the transistor can be regarded as being integrally formed.

The luminous brightness of the organic light-emitting element according to the present embodiment is controlled by a TFT, which is an example of a switching element, and a plurality of organic light-emitting elements arranged in a plane can display an image through the luminous brightness of each organic light-emitting element. The switching element according to the present embodiment is not limited to the TFT and may be a transistor formed of low-temperature polysilicon or an active-matrix driver formed on a substrate, such as a Si substrate. The expression "on a substrate" can also be referred to as "in a substrate". Whether a transistor is provided in a substrate or TFT is used is selected depending on the size of the display unit; for example, when the size is approximately 0.5 inches, an organic light-emitting element is preferably provided on a Si substrate.

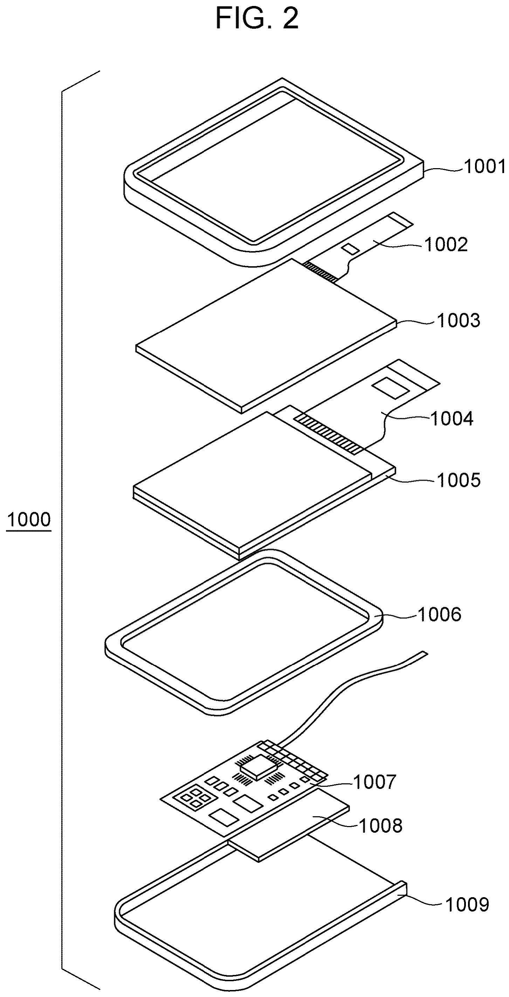

Fig. 2 is a schematic view of an example of the display apparatus according to the present embodiment. A display apparatus 1000 may include a touch panel 1003, a display panel 1005, a frame 1006, a circuit substrate 1007, and a battery 1008 between an upper cover 1001 and a lower cover 1009. The display panel 1005 may include the organic light-emitting element according to the present embodiment. The touch panel 1003 and the display panel 1005 are coupled to flexible print circuits FPC 1002 and 1004, respectively. A transistor is printed on the circuit substrate 1007. The battery 1008 may not be provided if the display apparatus is not a mobile device, or may be provided in another position even if the display apparatus is a mobile device.

The display apparatus according to the present embodiment may have red, green, and blue color filters. In the color filters, the red, green, and blue colors may be arranged in a delta arrangement.

The display apparatus according to the present embodiment may be used for a display unit of a mobile terminal. Such a display apparatus may have both a display function and an operation function. The mobile terminal may be a mobile phone, such as a smartphone, a tablet, a head-mounted display, or the like.

The display apparatus according to the present embodiment may be used for a display unit of an imaging apparatus including an image pickup element configured to receive light. The imaging apparatus may include a display unit that displays information acquired by the image pickup element. Furthermore, the display unit may be a display unit exposed to the outside of the imaging apparatus or a display unit disposed in a finder. The imaging apparatus may be a digital camera or a digital camcorder.

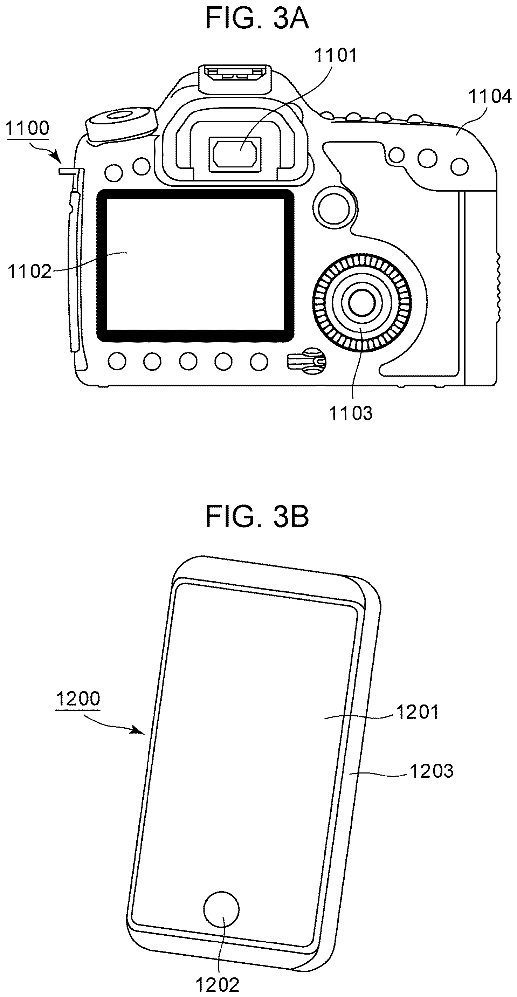

Fig. 3A is a schematic view of an example of an imaging apparatus according to the present embodiment. An imaging apparatus 1100 may include a viewfinder 1101, a rear display 1102, an operating unit 1103, and a housing 1104. The viewfinder 1101 and the rear display 1102 may include the organic light-emitting element according to the present embodiment. In such a case, the viewfinder 1101 and the rear display 1102 may display not only an image to be captured but also environmental information, an imaging instruction, and the like. The environmental information may include the intensity of external light, the direction of external light, the travel speed of a photographic subject, the possibility that the photographic subject is shielded by a shielding material, and the like.

Since the timing suitable for imaging is a short time, it is better to display the information as early as possible. Thus, a display apparatus including the organic light-emitting element according to the present embodiment is preferably used. This is because the organic light-emitting element has a high response speed.

The imaging apparatus 1100 may further include an optical unit (not shown). The optical unit may include a single lens or a plurality of lenses and focuses on the image pickup element in the housing 1104. The focus of the plurality of lenses can be adjusted by adjusting their relative positions. This operation can also be automatically performed. The imaging apparatus may be referred to as a photoelectric conversion apparatus. The photoelectric conversion apparatus can have, as an imaging method, a method of detecting a difference from a previous image, a method of cutting out a permanently recorded image, or the like, instead of taking an image one after another.

Fig. 3B is a schematic view of an example of electronic equipment according to the present embodiment. Electronic equipment 1200 includes a display unit 1201, an operating unit 1202, and a housing 1203. The housing 1203 may include a circuit, a printed circuit board including the circuit, a battery, and a communication unit. The operating unit 1202 may be a button or a touch panel type reaction unit. The operating unit may be a biometric recognition unit that recognizes a fingerprint for unlocking or the like. Electronic equipment with a communication unit may also be referred to as communication equipment. The electronic equipment may further include a lens and an image pickup element for a camera function. An image taken by the camera function is displayed on the display unit. The electronic equipment may be a smartphone, a notebook computer, or the like.

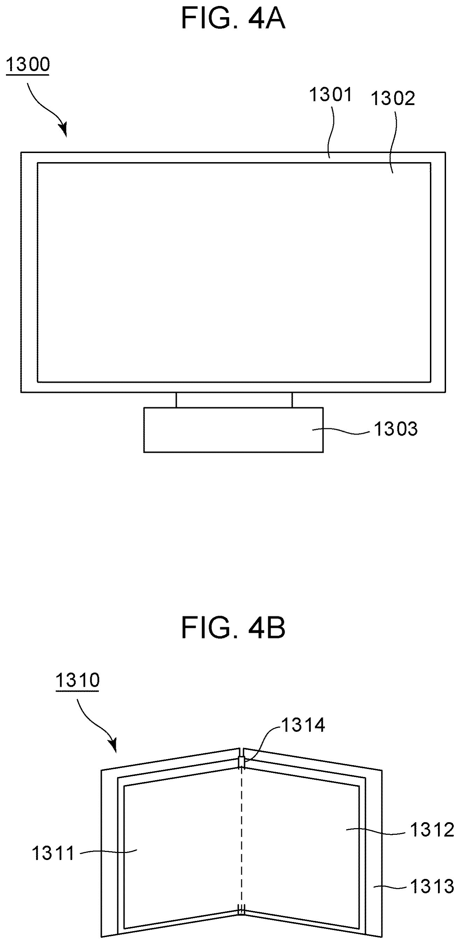

Figs. 4A and 4B are schematic views of an example of the display apparatus according to the present embodiment. Fig. 4A illustrates a display apparatus, such as a television monitor or a PC monitor. A display apparatus 1300 includes a housing 1301 and a display unit 1302. The organic light-emitting element according to the present embodiment may be used for the display unit 1302.

The display apparatus 1300 may include the housing 1301 and a base 1303 that supports the display unit 1302. The base 1303 is not limited to the form illustrated in Fig. 4A. The lower side of the housing 1301 may also serve as a base.

The housing 1301 and the display unit 1302 may be bent. The radius of curvature thereof may be 5000 mm or more and 6000 mm or less.

Fig. 4B is a schematic view of another example of the display apparatus according to the present embodiment. A display apparatus 1310 in Fig. 4B is configured to be foldable and is a so-called foldable display apparatus. The display apparatus 1310 includes a first display unit 1311, a second display unit 1312, a housing 1313, and a folding point 1314. The first display unit 1311 and the second display unit 1312 may include the organic light-emitting element according to the present embodiment. The first display unit 1311 and the second display unit 1312 may be one seamless display apparatus. The first display unit 1311 and the second display unit 1312 can be divided by a folding point. The first display unit 1311 and the second display unit 1312 may display different images or one image.

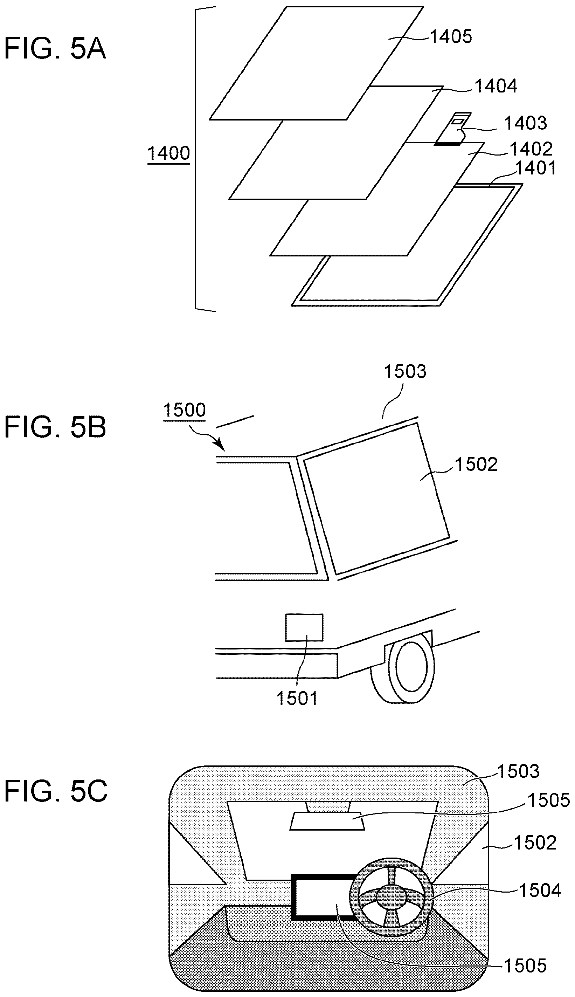

Fig. 5A is a schematic view of an example of a lighting apparatus according to the present embodiment. A lighting apparatus 1400 may include a housing 1401, a light source 1402, and a circuit substrate 1403. The light source 1402 may include the organic light-emitting element according to the present embodiment. The lighting apparatus 1400 may include an optical film 1404 to improve the color rendering properties of the light source. Furthermore, the lighting apparatus 1400 may include a light-diffusing unit 1405 to effectively diffuse light of the light source. The light-diffusing unit 1405 enables the lighting apparatus 1400 to distribute light over a wide area. The optical film 1404 and the light-diffusing unit 1405 may be provided on the light output side of the illumination. If necessary, a cover may be provided on the outermost side.

The lighting apparatus is, for example, an interior lighting apparatus. The lighting apparatus may emit white light, neutral white light, or light of any color from blue to red. The lighting apparatus according to the present embodiment may include a light modulating circuit for modulating light thereof. The lighting apparatus according to the present embodiment may include a power supply circuit coupled to the organic light-emitting element according to the present embodiment. The power supply circuit may be a circuit that converts an AC voltage into a DC voltage. The white has a color temperature of 4200 K, and the neutral white has a color temperature of 5000 K. The lighting apparatus according to the present embodiment may further include a color filter.

Furthermore, the lighting apparatus according to the present embodiment may include a heat dissipation unit. The heat dissipation unit dissipates heat in the apparatus to the outside of the apparatus and may be a metal, ceramic, or the like with a high thermal conductivity.

Fig. 5B is a schematic view of an automobile as an example of a movable body according to the present embodiment. The automobile includes a taillight, which is an example of a lamp. An automobile 1500 includes a taillight 1501 and an automotive body 1503 and may have a form in which the taillight is lit when a brake operation or the like is performed. The automotive body 1503 can also referred to as a body. The automobile 1500 may have a window 1502 on the automotive body 1503. The taillight 1501 may include the organic light-emitting element according to the present embodiment. The taillight may include a protective member that protects a light source. The protective member may be formed of any transparent material with moderately high strength and is preferably formed of polycarbonate or the like. The polycarbonate may be mixed with a furandicarboxylic acid derivative, an acrylonitrile derivative, or the like.

The window 1502 may be a transparent display, provided that the window 1502 is not a window for checking the front and rear of the automobile. The transparent display may include the organic light-emitting element according to the present embodiment. In such a case, a constituent material, such as an electrode, of the organic light-emitting element according to the present embodiment is composed of a transparent member.

As illustrated in Fig. 5C, the automobile 1500 includes a steering wheel 1504 for controlling the movement direction of the movable body, a display unit 1505 that displays a map, the position of the movable body, a turning direction, and the like and that is incorporated in the automotive body 1503, and the like. The display unit 1505 may include the organic light-emitting element according to the present embodiment.

The movable body according to the present embodiment includes one or both of a driving force generator that generates a driving force mainly used for the movement of the movable body and a rotatable body mainly used for the movement of the movable body. The driving force generator may be an engine, a motor, or the like. The rotatable body may be a tire, a wheel, a screw of a ship, or the like. More specifically, the movable body may be a bicycle, an automobile, a train, a ship, an aircraft, a drone, or the like. The movable body may include a body and a lamp provided on the body or a display unit provided on the body. The lamp may emit light to indicate the position of the body.

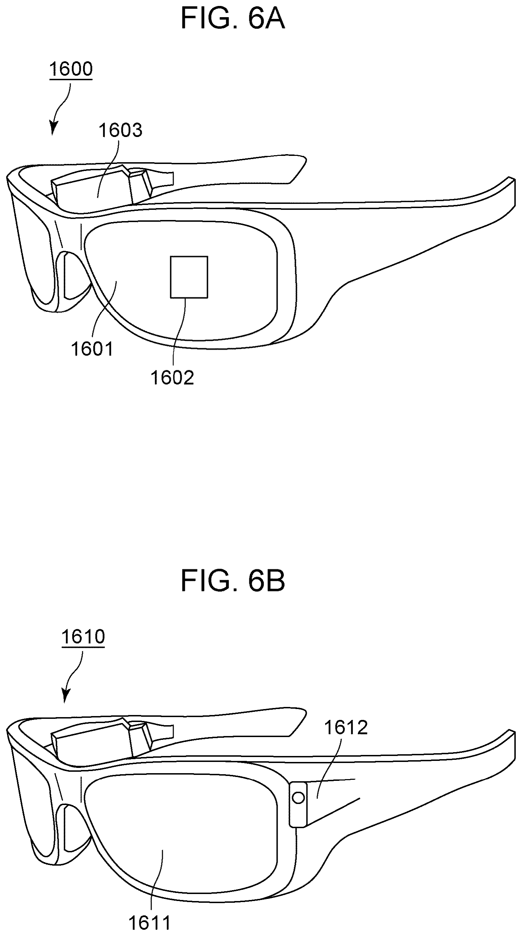

An application example of the display apparatus of each of the above-described embodiments will be described with reference to Figs. 6A and 6B. The display apparatus can be applied to, for example, a system that can be worn as a wearable device, such as smart glasses, a head-mounted display, or a smart contact lens. A display apparatus that can be used for a wearable device may include an imaging apparatus capable of photoelectrically converting visible light and a display apparatus capable of emitting visible light.

Figs. 6A and 6B are schematic views of an example of glasses (smart glasses) according to the present embodiment. Glasses 1600 (smart glasses) will be described with reference to Fig. 6A. The glasses 1600 include a display unit on the back side of a lens 1601. The display unit may include the organic light-emitting element according to the present embodiment. Furthermore, an imaging apparatus 1602, such as a CMOS sensor or SPAD, may be provided on the front side of the lens 1601.

The glasses 1600 further include a controller 1603. The controller 1603 functions as a power supply for supplying electric power to the imaging apparatus 1602 and the display unit. The controller 1603 controls the operation of the imaging apparatus 1602 and the display unit. The lens 1601 has an optical system for condensing light of the imaging apparatus 1602 and the display unit.

Glasses 1610 (smart glasses) will be described with reference to Fig. 6B. The glasses 1610 include a controller 1612, and the controller 1612 includes a display apparatus including the organic light-emitting element according to the present embodiment. The controller 1612 may further include an imaging apparatus corresponding to the imaging apparatus 1602. A lens 1611 includes an optical system for projecting light from the controller 1612, and an image is projected on the lens 1611. The controller 1612 functions as a power supply that supplies electric power to the imaging apparatus and the display apparatus, and controls the operation of the imaging apparatus and the display apparatus. The controller may include a line-of-sight detection unit that detects a line of sight of the wearer. Infrared radiation may be used to detect the line of sight. An infrared radiation unit emits infrared radiation to the eyeball of the user gazing at a display image. Of the emitted infrared light, infrared light reflected from the eyeball is detected by an imaging unit including a lightreceiving element to capture an image of the eyeball. A reduction unit for reducing light from the infrared radiation unit to a display unit in a plan view is provided to reduce degradation in image quality.

From the image of the eyeball captured by infrared imaging, the controller 1612 detects the line of sight of the user for the display image. Any known technique can be applied to line-of-sight detection using the captured image of the eyeball. As an example, a line-of-sight detection method can be used based on a Purkinje image due to reflection of irradiation light on the cornea.

More specifically, a line-of-sight detection process based on a pupil-corneal reflection method is performed. The line of sight of the user is detected by calculating a line-of-sight vector representing the direction (rotation angle) of the eyeball on the basis of an image of a pupil and a Purkinje image included in the captured image of the eyeball using the pupil-corneal reflection method.

The display apparatus according to the present embodiment may include an imaging apparatus including a light-receiving element and may control a display image on the basis of line-of-sight information of the user from the imaging apparatus.

More specifically, on the basis of the line-of-sight information, the display apparatus determines a first visibility region at which the user gazes and a second visibility region other than the first visibility region. The first visibility region and the second visibility region may be determined by the controller of the display apparatus or may be received from an external controller. In the display region of the display apparatus, the first visibility region may be controlled to have higher display resolution than the second visibility region. In other words, the second visibility region may have lower resolution than the first visibility region.

The display region includes the first visibility region and the second visibility region different from the first visibility region, and a region with a higher priority is determined from the first visibility region and the second visibility region based on the line-of-sight information. The first visibility region and the second visibility region may be determined by the controller of the display apparatus or may be received from an external controller. The region with a higher priority may be controlled to have higher resolution than a region other than the region with a higher priority. In other words, a region with a lower priority may have lower resolution.

The first visibility region or a visibility region with a higher priority may be determined by artificial intelligence (Al). The AI may be a model configured to estimate the angle of the line of sight and the distance to a target ahead of the line of sight from an image of the eyeball using the image of the eyeball and the direction in which the eyeball actually viewed in the image as teaching data. The AI may be included in the display apparatus, the imaging apparatus, or an external apparatus. In a case where the external apparatus includes the AI, the AI can be preferably applied to smart glasses further including an imaging apparatus that images the outside. Smart glasses can display captured external information in real time.

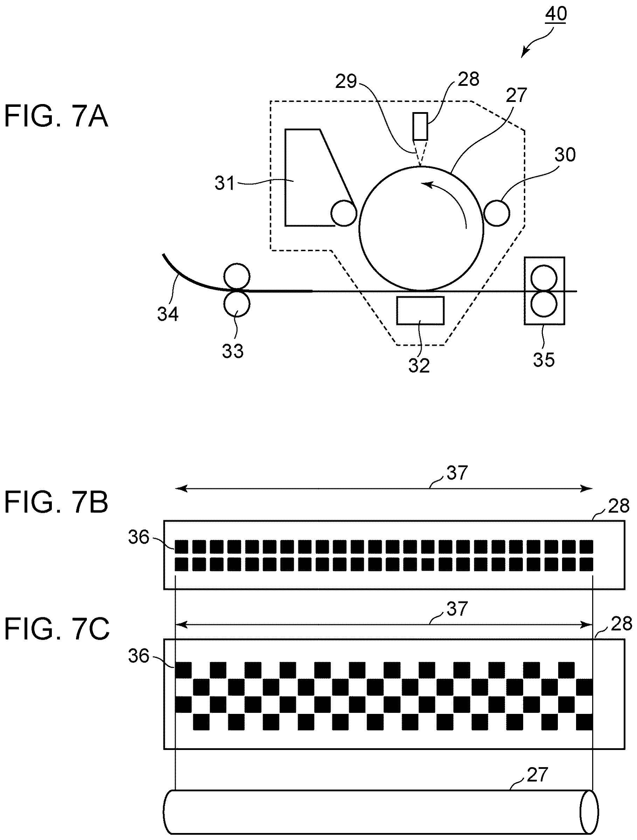

Fig. 7A is a schematic view of an example of an image-forming apparatus according to the present embodiment. An image-forming apparatus 40 is an electrophotographic image-forming apparatus and includes a photoconductor 27, an exposure light source 28, a charging unit 30, a development unit 31, a transfer unit 32, a feed roller 33, and a fixing unit 35. The exposure light source 28 emits light 29, and an electrostatic latent image is formed on the surface of the photoconductor 27. The exposure light source 28 may include the organic light-emitting element according to the present embodiment. The development unit 31 contains toner and the like. The charging unit 30 charges the photoconductor 27. The transfer unit 32 transfers a developed image to a recording medium 34. The feed roller 33 transports the recording medium 34. The recording medium 34 is, for example, paper. The fixing unit 35 fixes the image formed on the recording medium 34.

Figs. 7B and 7C are schematic views of the exposure light source 28, wherein a plurality of light-emitting portions 36 are arranged on a long substrate. An arrow 37 indicates a column direction in which the organic light-emitting elements are arranged. The column direction is the same as the direction of the axis about which the photoconductor 27 rotates. This direction can also be referred to as the major-axis direction of the photoconductor 27. Fig. 7B illustrates the light-emitting portions 36 arranged in the major-axis direction of the photoconductor 27. Unlike Fig. 7B, Fig. 7C illustrates the light-emitting portions 36 alternately arranged in the column direction in the first column and the second column. The first column and the second column are arranged at different positions in the row direction. In the first column, a plurality of light-emitting portions 36 are arranged at intervals. The second column has light-emitting portions 36 at positions corresponding to the intervals between the light-emitting portions 36 of the first column. That is, a plurality of light-emitting portions 36 are also arranged at intervals in the row direction. The arrangement in Fig. 7C can also be referred to as, for example, a grid-like pattern, a staggered pattern, or a checkered pattern.

As described above, the use of the apparatus including the organic light-emitting element according to the present embodiment enables stable display with high image quality even in long-term display.

### [Exemplary Embodiments]

The present embodiment will be described below with reference to exemplary embodiments. However, the present embodiment is not limited thereto.

### [Exemplary Embodiment 1 (Synthesis of Exemplary Compound A12)]

An acenaphtho[1,2-k]benzo[e]acephenanthrene skeleton was synthesized according to the following synthesis procedure with reference to the synthesis procedure described in Patent Literature 1.

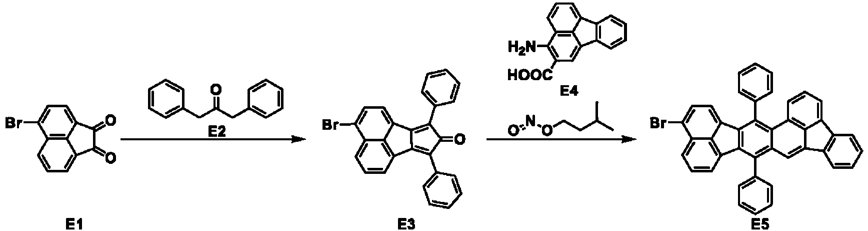

### (1) Synthesis of Compound E3

A 100-ml recovery flask was charged with the following reagent(s) and solvent(s).
Compound E1: 2.00 g (7.66 mmol)
Compound E2: 1.69 g (8.04 mmol)
Ethanol: 40 ml

Next, 556 mg (8.43 mmol) of 85% sodium hydroxide was dissolved in 10 ml of ethanol, and the solution was added dropwise into the recovery flask at room temperature. After completion of the dropwise addition, the product was heated to 40°C in a nitrogen stream and was stirred for 10 hours. After completion of the reaction, water was added to the reaction solution, the reaction solution was filtered, and the filtrate was dispersed and washed with water and methanol to produce 3.07 g of a dark green compound E3 (yield: 92%).

### (2) Synthesis of Compound E5

A 100-ml recovery flask was charged with the following reagent(s) and solvent(s).
Compound E3: 3.00 g (6.89 mmol)
Compound E4: 1.98 g (7.58 mmol)
Isoamyl nitrite: 1.02 ml (7.58 mmol)
Toluene: 50 ml

The reaction solution was then heated to 105°C in a nitrogen stream and was stirred for 2 hours. Furthermore, 669 mg (2.56 mmol) of the compound E4 and 0.34 ml (2.56 mmol) of isoamyl nitrite were added to the reaction solution, and the reaction solution was stirred for 2 hours. After completion of the reaction, extraction was performed using toluene and water, and the extract was concentrated and purified by silica gel column chromatography (heptane:toluene = 4:1) and was then dispersed and washed with heptane/ethanol to produce 1.41 g of a yellow compound E5 (yield: 47%).

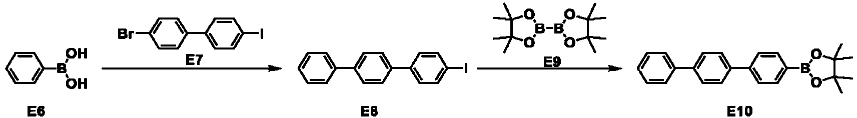

### (3) Synthesis of Compound E8

A 300-ml recovery flask was charged with the following reagent(s) and solvent(s).
Compound E6: 2.00 g (16.4 mmol)
Compound E7: 5.89 g (16.4 mmol)
Toluene: 60 ml
Ethanol: 30 ml
10% sodium carbonate aqueous solution: 30 ml
Tetrakis(triphenylphosphine)palladium: 94.7 mg (0.82 mmol)

The reaction solution was then heated to 90°C in a nitrogen stream and was stirred for 2 hours. After completion of the reaction, extraction was performed with toluene, and the extract was concentrated and purified by silica gel column chromatography (heptane:toluene mixture) to produce 5.51 g of a colorless compound E8 (yield: 87%).

### (4) Synthesis of Compound E10

A 200-ml recovery flask was charged with the following reagent(s) and solvent(s).
Compound E8: 2.00 g (6.47 mmol)
Compound E9: 1.64 g (6.47 mmol)
Pd(OAc)₂: 72.6 mg (0.32 mmol)
SPhos: 63.4 mg (0.65 mmol)
KOAc: 3.32 g (33.9 mmol)
Toluene: 60 ml

The reaction solution was then heated to 90°C in a nitrogen stream and was stirred for 3 hours. After completion of the reaction, extraction was performed with toluene, and the extract was concentrated and purified by silica gel column chromatography (toluene) to produce 1.73 g of a colorless compound E10 (yield: 75%).

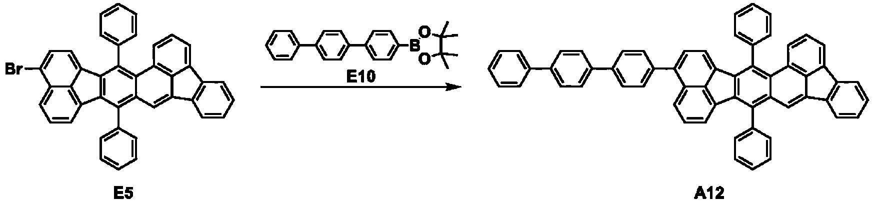

### (5) Synthesis of Compound A12

A 100-ml recovery flask was charged with the following reagent(s) and solvent(s).
Compound E5: 0.5 g (0,82 mmol)
Compound E10: 0,29 g (0.83 mmol)
Pd(OAc)₂: 9.20 mg (0.04 mmol)
SPhos: 33.6 mg (0.082 mmol)
KOAc: 0.40 g (4.10 mmol)
Toluene: 20 ml

The reaction solution was then heated to 90°C in a nitrogen stream and was stirred for 3 hours. After completion of the reaction, extraction was performed with toluene, and the extract was concentrated and purified by silica gel column chromatography (heptane:toluene mixture) to produce 0.34 g of a yellow compound A11 (yield: 55%).

The exemplary compound A11 was subjected to mass spectrometry using MALDI-TOF-MS (Autoflex LRF manufactured by Bruker).
[MALDI-TOF-MS] Measured value: m/z=756 Calculated value: C₆₀H₃₆ = 756 (Synthesis of exemplary Compound D27)

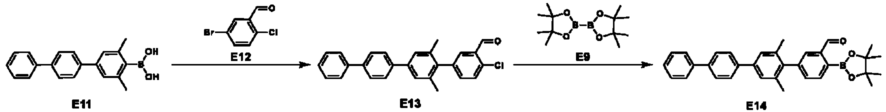

### (6) Synthesis of Compound E13

A 300-ml three-neck flask was charged with the following reagent(s) and solvent(s).
Compound E11: 10.00 g (0.05mol)
Compound E12: 13.2 g (0.06mol)
1,2-dimethoxyethane: 100 ml
2 M aqueous sodium carbonate: 40 ml
Tetrakis(triphenylphosphine)palladium: 2.88 g (5.0 mmol)

The reaction solution was then heated to 90°C in a nitrogen stream and was stirred for 7 hours. After completion of the reaction, extraction was performed with toluene, and the extract was concentrated to produce 18.8 g of a compound E13 (yield: 95%).

### (7) Synthesis of Compound E14

A 500-ml three-neck flask was charged with the following reagent(s) and solvent(s).
Compound E13: 13.00 g (0.03mol)
Compound E9: 15.6 g (0.06mol)
Pd(OAc)₂: 0.90 g (0.4 mmol)
SPhos: 0.49 g (1.2 mmol)
KOAc: 3.32 g (0.12mol)
Toluene: 300 ml

The reaction solution was then heated to 90°C in a nitrogen stream and was stirred for 5 hours. After completion of the reaction, the product was filtered through silica gel and was washed with toluene to produce 12.8 g of a compound E14 (yield: 88%).

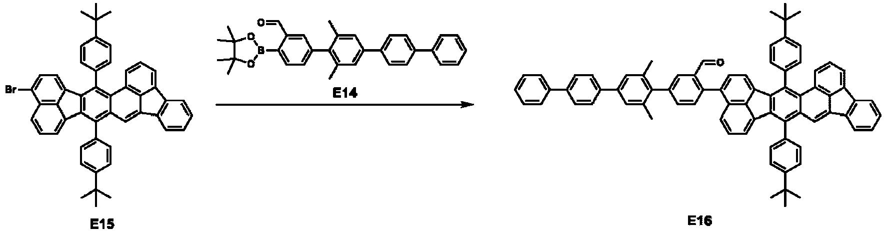

### (8) Synthesis of Compound E16

A 500-ml three-neck flask was charged with the following reagent(s) and solvent(s).
Compound E15: 2.00 g (2.78 mmol)
Compound E14: 2.04 g (4.17 mmol)
Toluene: 100 ml
2 M aqueous sodium carbonate: 10 ml
Tetrakis(triphenylphosphine)palladium: 0.19 g (0.14 mmol)

The reaction solution was then heated to 90°C in a nitrogen stream and was stirred for 15 hours. After completion of the reaction, the product was filtered through silica gel and was washed with toluene. The product was then concentrated, was suspended and washed with methanol, and was filtered to produce 1.89 g of a yellow compound E16 (yield: 68%).

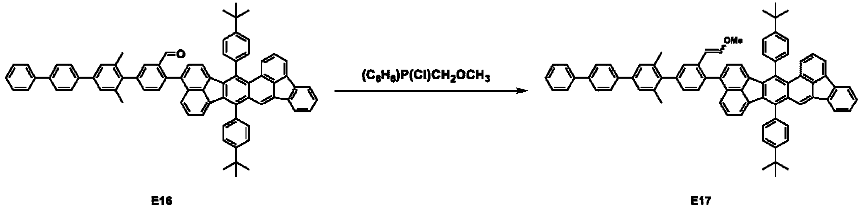

### (9) Synthesis of Compound E17

A 500-ml three-neck flask was charged with the following reagent(s) and solvent(s). The reaction was performed in a nitrogen atmosphere.
(Methoxymethyl)triphenylphosphonium chloride: 0.48 g (2.53 mmol)
THF (dehydrated): 75 mL

1.0 M/L t-BuOK/THF: 0.28 g (2.53 mmol)/2.53 mL THF was then added, and the product was stirred for 2 hours.

1.69 g (1.69 mmol) of the compound E15 in 75 mL of THF (dehydrated) was added, and the product was heated with stirring for 2 hours.

Water was added for quenching, followed by extraction with toluene. An organic layer was concentrated, and the resulting residue was purified by silica gel column chromatography (heptane:toluene mixture). The resulting solid was suspended and washed with methanol and was filtered to produce 1.56 g of a yellow compound E16 (yield: 90%).

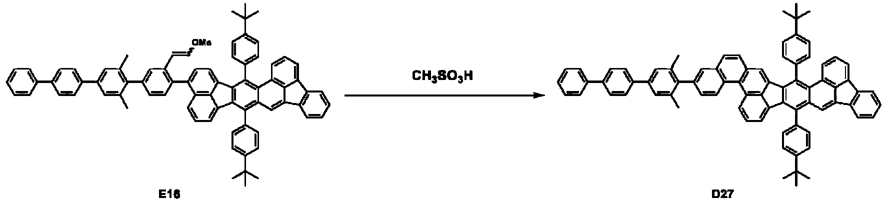

### (10) Synthesis of Compound D27

A 500-ml three-neck flask was charged with the following reagent(s) and solvent(s). The reaction was performed in a nitrogen atmosphere.
Compound E16: 1.5 g (1.5 mmol)
Dichloromethane: 50 ml

0.14 g (1.5 mmol) of methanesulfonic acid was added dropwise thereto, and the product was stirred for 1 hour. Aqueous sodium hydrogen carbonate was added for quenching, and the product was then subjected to extraction with dichloromethane. An organic layer was concentrated and purified by silica gel chromatography (heptane:toluene mixture). The resulting solid was suspended and washed with methanol and was filtered to produce 1.14 g of a yellow compound A10 (yield: 76%).

The exemplary compound A10 was subjected to mass spectrometry using MALDI-TOF-MS (Autoflex LRF manufactured by Bruker).
[MALDI-TOF-MS] Measured value: m/z=997 Calculated value: C₇₈H₆₀ = 997
[Exemplary Embodiments 2 to 63 (Synthesis of Exemplary Compounds)]

Exemplary compounds of Exemplary Embodiments 2 to 63 were synthesized in the same manner as in Exemplary Embodiment 1 except that, as shown in Table 6, the raw material E5 was changed to a raw material 1 and the raw material E10 was changed to a raw material 2.

Measured values m/z measured by mass spectrometry in the same manner as in Exemplary Embodiment 1 are also shown.

**Table 6-1**

| Exemplary embodiment | Exemplary compound | Raw material 1 | Raw material 2 | m/z |
|---|---|---|---|---|
| 2 | A1 | 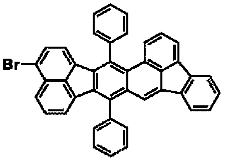 | 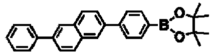 | 807 |
| 3 | A2 | 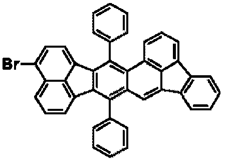 | 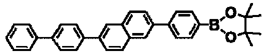 | 883 |
| 4 | A3 | 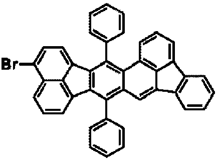 | 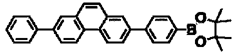 | 856 |
| 5 | A4 | 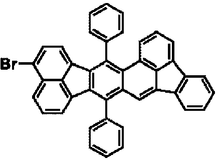 | 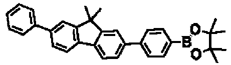 | 872 |
| 6 | A5 | 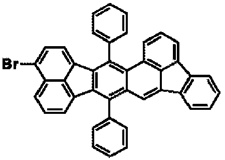 | 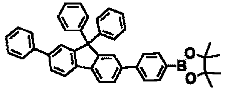 | 996 |
| 7 | A6 | 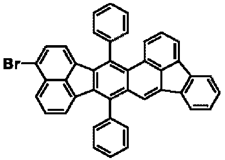 | 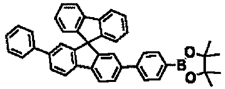 | 994 |
| 8 | A7 | 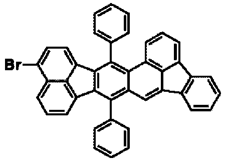 | 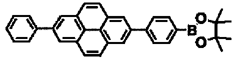 | 880 |

**Table 6-2**

| Exemplary embodiment | Exemplary compound | Raw material 1 | Raw material 2 | m/z |
|---|---|---|---|---|
| 9 | A8 | 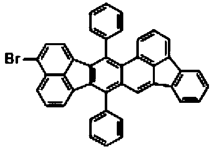 | 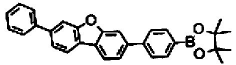 | 846 |
| 10 | A9 | 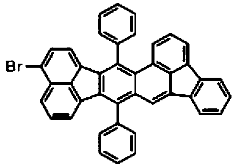 | 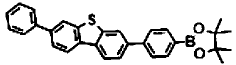 | 862 |
| 11 | A11 | 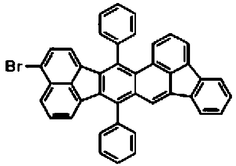 | 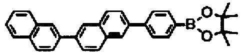 | 856 |
| 12 | A12 | 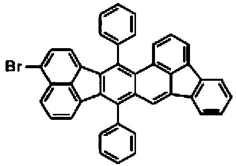 | 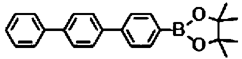 | 756 |
| 13 | A13 | 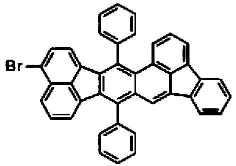 | 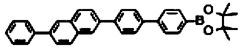 | 883 |
| 14 | A14 | 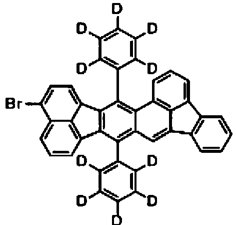 | 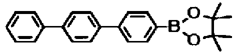 | 766 |

**Table 6-3**

| Exemplary embodiment | Exemplary compound | Raw material 1 | Raw material 2 | m/z |
|---|---|---|---|---|
| 16 | A26 | 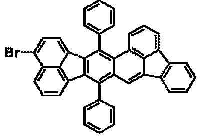 | 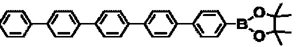 | 888 |
| 17 | A29 | 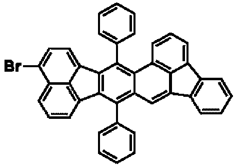 | 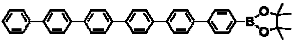 | 908 |
| 18 | A31 | 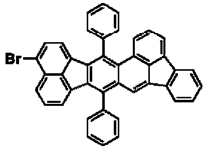 | 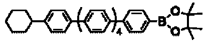 | 1066 |
| 19 | A51 | 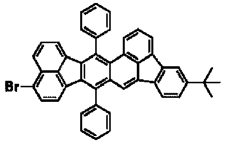 | 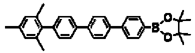 | 930 |
| 20 | B1 | 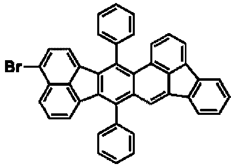 | 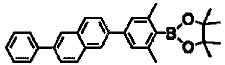 | 758 |
| 21 | B2 | 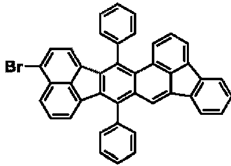 | 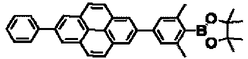 | 908 |
| 22 | B3 | 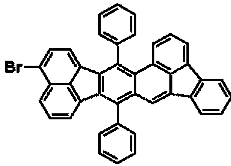 | 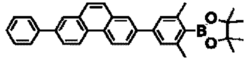 | 884 |

**Table 6-4**

| Exemplary embodiment | Exemplary compound | Raw material 1 | Raw material 2 | m/z |
|---|---|---|---|---|
| 23 | B4 | 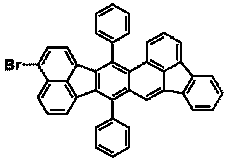 | 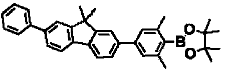 | 900 |
| 24 | B5 | 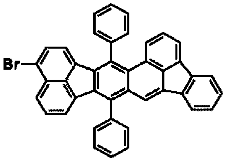 | 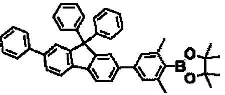 | 1024 |
| 25 | B6 | 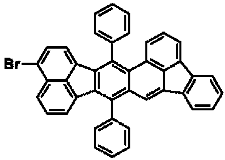 | 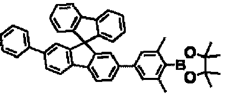 | 1022 |
| 26 | B7 | 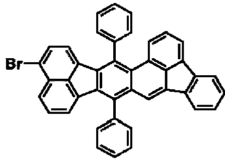 | 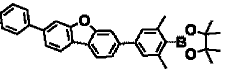 | 874 |
| 27 | B8 | 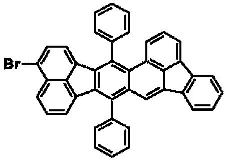 | 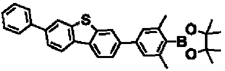 | 890 |
| 28 | B10 | 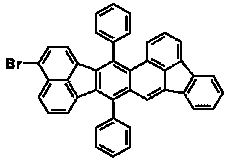 | 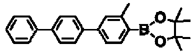 | 770 |
| 29 | B11 | 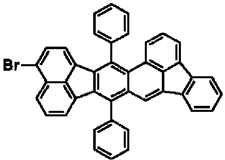 | 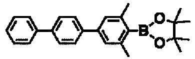 | 784 |

**Table 6-5**

| Exemplary embodiment | Exemplary compound | Raw material 1 | Raw material 2 | m/z |
|---|---|---|---|---|
| 30 | B12 | 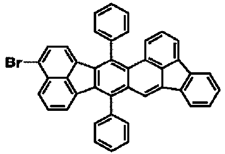 | 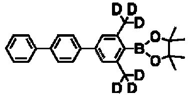 | 790 |
| 31 | B13 | 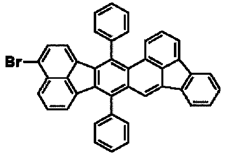 | 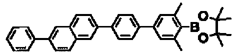 | 911 |
| 32 | B14 | 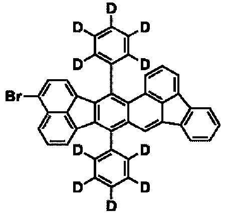 | 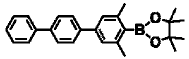 | 794 |
| 33 | B19 | 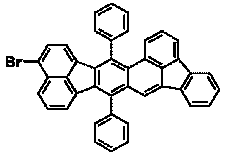 | 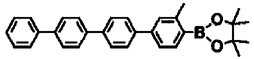 | 847 |
| 34 | B20 | 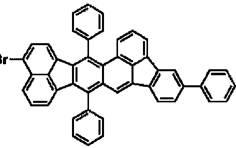 | 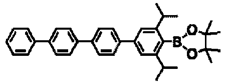 | 992 |
| 35 | B22 | 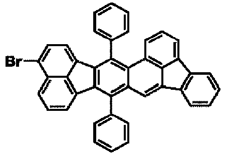 | 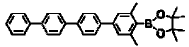 | 861 |
| 36 | B25 | 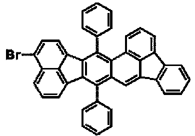 | 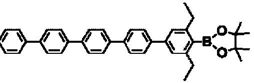 | 889 |

**Table 6-6**

| Exemplary embodiment | Exemplary compound | Raw material 1 | Raw material 2 | m/z |
|---|---|---|---|---|
| 37 | B26 | 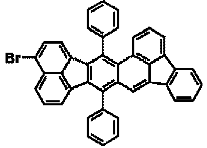 | 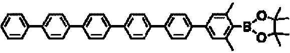 | 1012 |
| 38 | B29 | 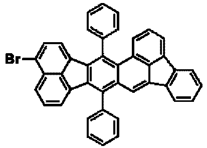 | 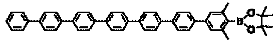 | 1088 |
| 39 | B32 | 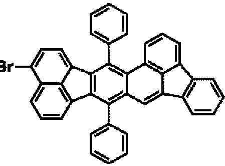 | 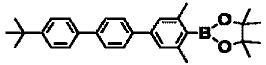 | 841 |
| 40 | B42 | 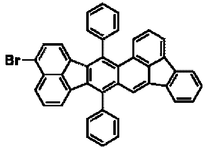 | 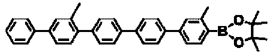 | 936 |
| 41 | C1 | 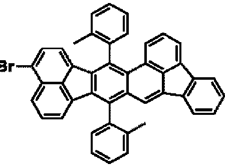 | 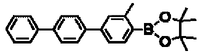 | 799 |
| 42 | C2 | 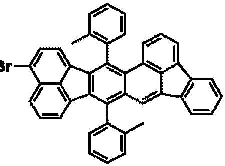 | 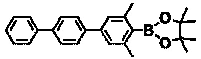 | 813 |
| 43 | C3 | 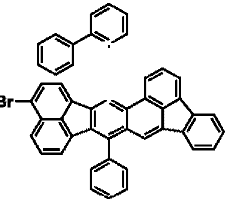 | 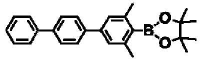 | 861 |

**Table 6-7**

| Exemplary embodiment | Exemplary compound | Raw material 1 | Raw material 2 | m/z |
|---|---|---|---|---|
| 44 | C4 | 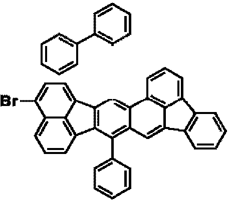 | 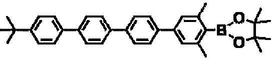 | 993 |
| 45 | C5 | 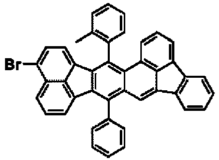 | 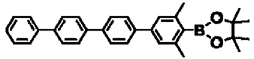 | 861 |
| 46 | C7 | 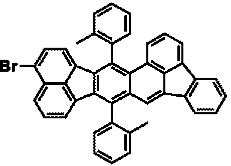 | 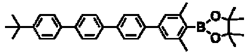 | 965 |
| 47 | C8 | 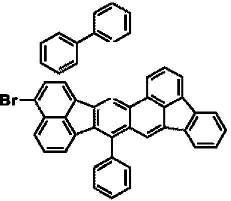 | 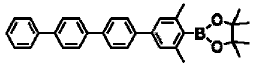 | 937 |
| 48 | C12 | 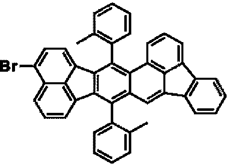 | 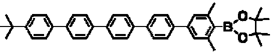 | 1021 |
| 49 | C24 | 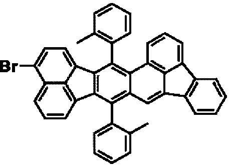 | 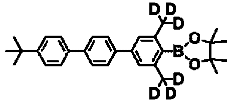 | 875 |

**Table 6-8**

| Exemplary embodiment | Exemplary compound | Raw material 1 | Raw material 2 | m/z |
|---|---|---|---|---|
| 50 | C32 | 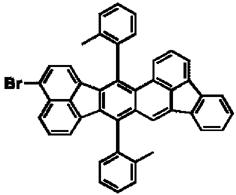 | 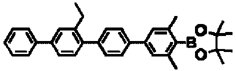 | 917 |
| 51 | C33 | 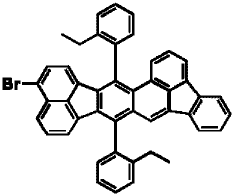 | 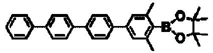 | 917 |
| 52 | C37 | 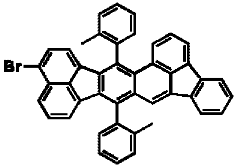 | 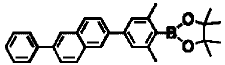 | 863 |
| 53 | C38 | 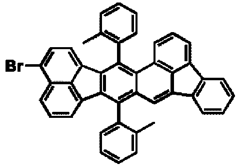 | 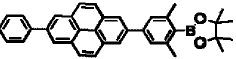 | 937 |
| 54 | C39 | 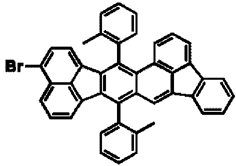 | 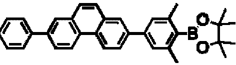 | 913 |
| 55 | C46 | 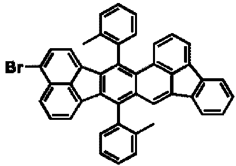 | 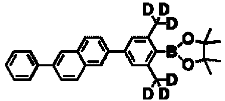 | 869 |

**Table 6-9**

| Exemplary embodiment | Exemplary compound | Raw material 1 | Raw material 2 | m/z |
|---|---|---|---|---|
| 56 | C56 | 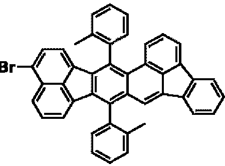 | 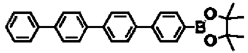 | 861 |
| 57 | D4 | 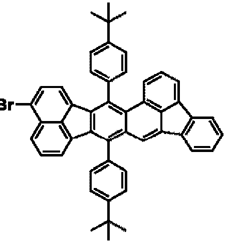 | 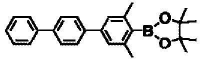 | 897 |
| 58 | D5 | 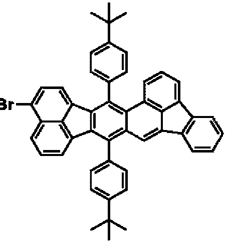 | 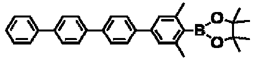 | 973 |
| 59 | D7 | 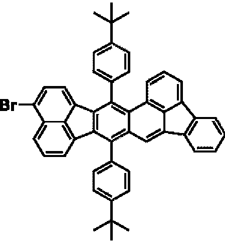 I | 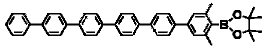 | 1125 |
| 60 | D8 | 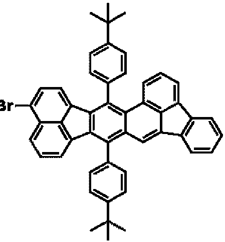 | 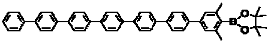 | 1201 |

**Table 6-10**

| Exemplary embodiment | Exemplary compound | Raw material 1 | Raw material 2 | m/z |
|---|---|---|---|---|
| 61 | D9 | 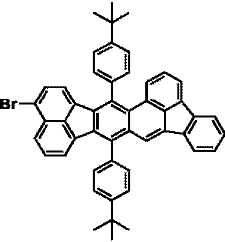 | 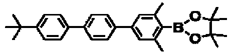 | 953 |
| 62 | D14 | 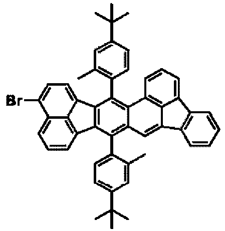 | 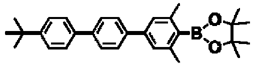 | 981 |
| 63 | D22 | 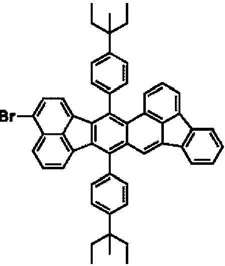 | 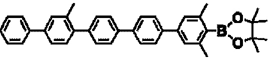 | 1119 |

### [Exemplary Embodiment 64]

In the present exemplary embodiment, an organic EL element of a bottom emission type structure was produced in which a positive electrode, a hole injection layer, a hole transport layer, an electron-blocking layer, a light-emitting layer, a hole-blocking layer, an electron transport layer, an electron injection layer, and a negative electrode were sequentially formed on a substrate.

First, an ITO film was formed on a glass substrate and was subjected to desired patterning to form an ITO electrode (positive electrode). The ITO electrode had a thickness of 100 nm. The substrate on which the ITO electrode was thus formed was used as an ITO substrate in the following steps. An organic compound layer and an electrode layer shown in Table 7 were then continuously formed on the ITO substrate by performing vacuum deposition by resistance heating in a vacuum chamber. At this time, the opposing electrodes (a metal electrode layer, a negative electrode) had an electrode area of 3 mm².

**Table 7**

| | Material | | | Film thickness (nm) |
|---|---|---|---|---|
| Negative electrode | Al | | | 100 |
| Electron injection layer (EIL) | LiF | | | 1 |
| Electron transport layer (ETL) | ET2 | | | 15 |
| Hole-blocking layer (HBL) | ET12 | | | 15 |
| Light-emitting layer (EML) | Host | EM2 | Mass ratio | 20 |
| | Guest | A12 | EM2:A12=9:1 | |
| Electron-blocking layer (EBL) | HT10 | | | 15 |
| Hole transport layer (ETL) | HT3 | | | 30 |
| Hole injection layer (HIL) | HT16 | | | 5 |

The characteristics of the element were measured and evaluated. Assuming that the efficiency ratio of Comparative Example 1 was 1.0, the maximum current efficiency of the light-emitting element was 1.1 in terms of an efficiency ratio. The emission spectrum of Comparative Example 1 when the brightness of the light-emitting element was 1000 cd/m2 was confirmed to be 460 nm. With respect to measuring apparatuses, more specifically, the current-voltage characteristics were measured with a microammeter 4140B manufactured by Hewlett-Packard Co., and the luminous brightness was measured with BM7 manufactured by Topcon Corporation.

### [Exemplary Embodiments 65 to 87, Comparative Example 1 and Comparative Example 2]

Organic light-emitting elements according to Exemplary Embodiments 65 to 87 and Comparative Examples 1 and 2 were produced in the same manner as in Exemplary Embodiment 64 except that the compounds shown in Table 8 were appropriately used. The characteristics of the elements were measured and evaluated in the same manner as in Exemplary Embodiment 64. Table 8 shows the measurement results.

A and B in the table are as follows:
A: The emission wavelength was 455 nm or less.
B: The emission wavelength was 456 nm or more and less than 460 nm.
C: The emission wavelength was 460 nm or more.

**Table 8**

| | HIL | HTL | EBL | EML | | HBL | ETL | Efficiency ratio | Emission wavelength |
|---|---|---|---|---|---|---|---|---|---|
| | | | | Host | Guest | | | | |
| Exemplary embodiment 65 | HT16 | HT3 | HT10 | EM2 | A2 | ET11 | ET2 | 1.1 | B |
| Exemplary embodiment 66 | HT16 | HT2 | HT10 | EM2 | A12 | ET11 | ET2 | 1.2 | B |
| Exemplary embodiment 67 | HT16 | HT3 | HT12 | EM2 | A12 | ET12 | ET3 | 1.1 | B |
| Exemplary embodiment 68 | HT16 | HT3 | HT12 | EM2 | A26 | ET12 | ET3 | 1.1 | B |
| Exemplary embodiment 69 | HT16 | HT3 | HT12 | EM2 | B2 | ET11 | ET7 | 1.4 | A |
| Exemplary embodiment 70 | HT16 | HT3 | HT10 | EM2 | B10 | ET11 | ET2 | 1.3 | A |
| Exemplary embodiment 71 | HT16 | HT3 | HT10 | EM4 | B22 | ET11 | ET2 | 1.3 | A |
| Exemplary embodiment 72 | HT16 | HT7 | HT12 | EM2 | B22 | ET11 | ET2 | 1.3 | A |
| Exemplary embodiment 73 | HT16 | HT3 | HT12 | EM2 | B32 | ET11 | ET7 | 1.4 | A |
| Exemplary embodiment 74 | HT17 | HT3 | HT10 | EM2 | C2 | ET11 | ET2 | 1.2 | A |
| Exemplary embodiment 75 | HT16 | HT3 | HT12 | EM2 | C4 | ET11 | ET7 | 1.4 | A |
| Exemplary embodiment 76 | HT16 | HT3 | HT12 | EM2 | C7 | ET12 | ET7 | 1.4 | A |
| Exemplary embodiment 77 | HT16 | HT3 | HT12 | EM26 | C7 | ET11 | ET7 | 1.4 | A |
| Exemplary embodiment 78 | HT16 | HT3 | HT10 | EM2 | C24 | ET11 | ET2 | 1.3 | A |
| Exemplary embodiment 79 | HT16 | HT3 | HT10 | EM26 | C24 | ET11 | ET7 | 1.3 | A |
| Exemplary embodiment 80 | HT16 | HT3 | HT10 | EM4 | C37 | ET11 | ET2 | 1.3 | A |
| Exemplary embodiment 81 | HT16 | HT3 | HT10 | EM26 | C56 | ET11 | ET7 | 1.3 | A |
| Exemplary embodiment 82 | HT16 | HT3 | HT12 | EM2 | D4 | ET12 | ET2 | 1.4 | A |
| Exemplary embodiment 83 | HT16 | HT3 | HT12 | EM2 | D5 | ET12 | ET2 | 1.4 | A |
| Exemplary embodiment 84 | HT17 | HT3 | HT10 | EM2 | D5 | ET11 | ET2 | 1.6 | A |
| Exemplary embodiment 85 | HT17 | HT3 | HT10 | EM2 | D9 | ET11 | ET2 | 1.4 | A |
| Exemplary embodiment 86 | HT16 | HT3 | HT12 | EM4 | D9 | ET11 | ET2 | 1.5 | A |
| Exemplary embodiment 87 | HT17 | HT3 | HT12 | EM2 | D14 | ET12 | ET2 | 1.6 | A |
| Exemplary embodiment 88 | HT17 | HT7 | HT10 | EM4 | D27 | ET12 | ET7 | 1.2 | B |
| Comparative example 1 | HT16 | HT3 | HT10 | EM2 | Comparative compound 1-a | ET11 | ET2 | 1.0 | B |
| Comparative example 2 | HT16 | HT3 | HT10 | EM2 | Comparative compound 2-a | ET11 | ET2 | 1.0 | C |

Table 8 showed that the organic light-emitting elements according to the exemplary embodiments had higher light emission efficiency and shorter light emission wavelength than the organic light-emitting elements according to the comparative examples. This is probably because a specific substituent provided in the major-axis direction in the organic compounds according to the present embodiment, which are guest materials, can achieve both an improvement in horizontal orientation property anad a reduction in extension of π conjugation.

For the above reasons, the organic compounds according to the present embodiment are organic compounds with high light emission efficiency and color purity.

The present embodiment may have the following configurations.

### (Configuration 1)

An organic compound represented by the general formula [1].

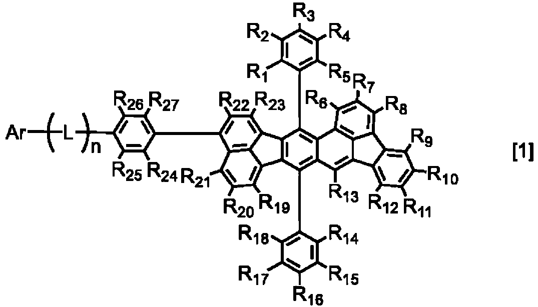

In the general formula [1], R₁ to R₂₇ are each independently selected from the group consisting of a hydrogen atom, a deuterium atom, a substituted or unsubstituted alkyl group, a substituted or unsubstituted alkoxy group, a substituted or unsubstituted aryl group, a substituted or unsubstituted aryloxy group, a substituted or unsubstituted heteroaryl group, a substituted or unsubstituted heteroaryloxy group, a substituted or unsubstituted amino group, and a cyano group. At least one of a pair of R₂₁ and R₂₄ and a pair of R₂₂ and R₂₇ may be bonded to each other to form a ring.

Ar denotes a substituted or unsubstituted aryl group.

n denotes an integer of 1 or more and 5 or less.

L denotes any one of the general formulae [2] to [7] or a combination thereof. When n denotes an integer of 2 or more, a plurality of Ls may be the same or different.

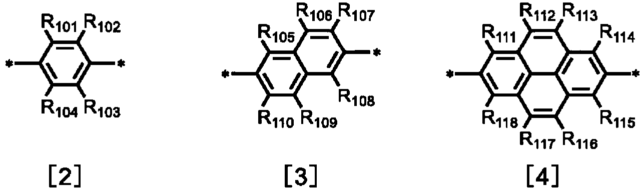

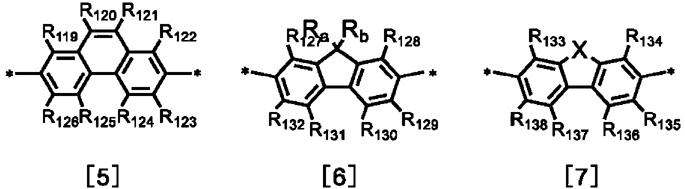

In the general formulae [2] to [7], R₁₀₁ to R₁₃₈, Rₐ, and R_{b} are each independently selected from a hydrogen atom, a substituted or unsubstituted alkyl group, and a substituted or unsubstituted aryl group. Rₐ and R_{b} may be bonded to each other to form a ring. X denotes an oxygen atom or a sulfur atom. * denotes a binding position.

### (Configuration 2)

The organic compound according to Configuration 1, wherein, in the general formula [1], R₁ to R₂₇ are each independently selected from the group consisting of a hydrogen atom, a deuterium atom, a substituted or unsubstituted alkyl group, a substituted or unsubstituted aryl group, and a substituted or unsubstituted heteroaryl group.

### (Configuration 3)

The organic compound according to Configuration 1 or 2, wherein, in the general formula (1), R₁ to R₂₇ are each independently selected from the group consisting of a hydrogen atom, a deuterium atom, a substituted or unsubstituted alkyl group having 1 or more and 6 or less carbon atoms, and a substituted or unsubstituted aryl group having 6 or more and 12 or less carbon atoms.

### (Configuration 4)

The organic compound according to any one of Configurations 1 to 3, wherein, in the general formula (1), R₁ to R₂₇ are each independently selected from the group consisting of a hydrogen atom, a deuterium atom, a methyl group, a CD₃ group, an isopropyl group, a tert-butyl group, C(CH₃)₂(C₂H₅), C(CH₃)(C₂H₅)₂, and a phenyl group.

### (Configuration 5)

The organic compound according to any one of Configurations 1 to 4, wherein, in the general formula (1), Ar denotes a substituted or unsubstituted aryl group having 6 or more and 20 or less carbon atoms.

### (Configuration 6)

The organic compound according to any one of Configurations 1 to 5, wherein, in the general formula (1), Ar denotes a substituted or unsubstituted aryl group having 6 or more and 12 or less carbon atoms.

### (Configuration 7)

The organic compound according to any one of Configurations 1 to 6, wherein, in the general formula (1), at least one of R₁, R₃, R₅, R₁₁, R₁₄, R₁₆, R₁₈, R₂₄, and R₂₇ is other than a hydrogen atom.

### (Configuration 8)

The organic compound according to any one of Configurations 1 to 7, wherein, in the general formula (1), at least one of R₂₄ and R₂₇ denotes a substituted or unsubstituted alkyl group.

### (Configuration 9)

The organic compound according to any one of Configurations 1 to 8, wherein, in the general formula (1), R₂₄ and R₂₇ denote a methyl group.

### (Configuration 10)

The organic compound according to any one of Configurations 1 to 9, wherein, in the general formula (1), at least one of R₁, R₅, R₁₄, and R₁₈ denotes a substituted or unsubstituted alkyl group or a substituted or unsubstituted aryl group.

### (Configuration 11)

The organic compound according to any one of Configurations 1 to 10, wherein, in the general formula (1), at least one of R₁, R₅, R₁₄, and R₁₈ denotes a methyl group or a phenyl group.

### (Configuration 12)

The organic compound according to any one of Configurations 1 to 11, wherein, in the general formula (1), at least one of R₃ and R₁₆ denotes a substituted or unsubstituted alkyl group.

### (Configuration 13)

An organic light-emitting element including:
a first electrode and a second electrode, and
an organic compound layer between the first electrode and the second electrode,
wherein the organic compound layer contains the organic compound according to one of Configurations 1 to 12.

### (Configuration 14)

The organic light-emitting element according to Configuration 13, wherein
the organic compound layer includes a light-emitting layer, and
the light-emitting layer contains the organic compound.

### (Configuration 15)

The organic light-emitting element according to Configuration 14, wherein
the light-emitting layer further contains a first compound, and
the first compound has a higher lowest singlet excitation energy than the organic compound.

### (Configuration 16)

The organic light-emitting element according to Configuration 15, wherein the first compound is composed of a hydrocarbon compound.

### (Configuration 17)

The organic light-emitting element according to Configuration 15, wherein
the light-emitting layer further contains a second compound, and
the second compound has a higher lowest singlet excitation energy than the organic compound and a lower lowest singlet excitation energy than the first compound.

### (Configuration 18)

A display apparatus including a plurality of pixels, wherein at least one of the plurality of pixels includes the organic light-emitting element according to any one of Configurations 13 to 17 and a transistor coupled to the organic light-emitting element.

### (Configuration 19)

A photoelectric conversion apparatus including:
an image pickup element configured to receive light; and
a display unit configured to display an image captured by the image pickup element,
wherein the display unit includes the organic light-emitting element according to any one of Configurations 13 to 17.

### (Configuration 20)

An image display apparatus including:
a display unit including the organic light-emitting element according to any one of Configurations 13 to 17; and
a housing in which the display unit is provided.

### (Configuration 21)

Electronic equipment including:
a display unit including the organic light-emitting element according to any one of Configurations 13 to 17;
a housing in which the display unit is provided; and
a communication unit that is provided in the housing and communicates with the outside.

### (Configuration 22)

A wearable device including:
a display unit including the organic light-emitting element according to any one of Configurations 13 to 17;
an optical system configured to condense light of the display unit; and
a controller configured to control display of the display unit.

### (Configuration 23)

A lighting apparatus including:
a light source including the organic light-emitting element according to any one of Configurations 13 to 17; and
a housing in which the light source is provided.

### (Configuration 24)

A movable body including:
a lamp including the organic light-emitting element according to any one of Configurations 13 to 17; and
a body provided with the lamp.

The present embodiment can provide an organic compound with high light emission efficiency and color purity.

Various embodiments have been described in detail above but it will be understood that the present disclosure is not limited to these embodiments and encompasses all modifications, variants, alternatives and equivalents falling within the scope of the appended claims.

## Claims

1. An organic compound represented by formula [1]: 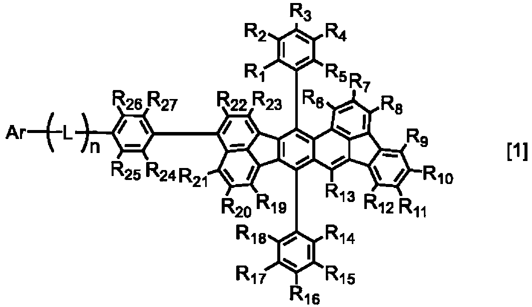
wherein R₁ to R₂₇ are each independently selected from the group consisting of a hydrogen atom, a deuterium atom, a substituted or unsubstituted alkyl group, a substituted or unsubstituted alkoxy group, a substituted or unsubstituted aryl group, a substituted or unsubstituted aryloxy group, a substituted or unsubstituted heteroaryl group, a substituted or unsubstituted heteroaryloxy group, a substituted or unsubstituted amino group, and a cyano group, at least one of a pair of R₂₁ and R₂₄ and a pair of R₂₂ and R₂₇ are optionally bonded to each other to form a ring,
Ar denotes a substituted or unsubstituted aryl group,
n denotes an integer of 1 or more and 5 or less,
L denotes any one of formulae [2] to [7] or a combination thereof, in a case that n denotes an integer of 2 or more, each L is independently selected from any one of formula [2] to [7], 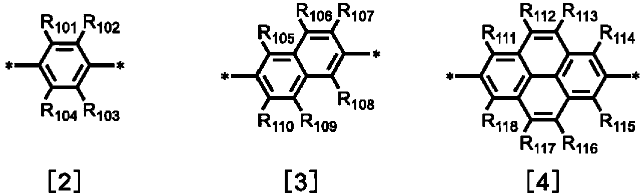 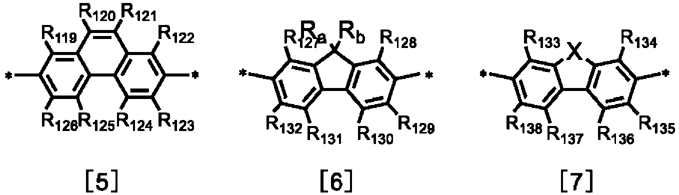
in formulae [2] to [7], R₁₀₁ to R₁₃₈, Rₐ, and R_{b} are each independently selected from a hydrogen atom, a substituted or unsubstituted alkyl group, and a substituted or unsubstituted aryl group, Rₐ and R_{b} are optionally bonded to each other to form a ring, X denotes an oxygen atom or a sulfur atom, and * denotes a binding position.

2. The organic compound according to claim 1, wherein
in formula [1], R₁ to R₂₇ are each independently selected from the group consisting of a hydrogen atom, a deuterium atom, a substituted or unsubstituted alkyl group, a substituted or unsubstituted aryl group, and a substituted or unsubstituted heteroaryl group,
optionally R₁ to R₂₇ are each independently selected from the group consisting of a hydrogen atom, a deuterium atom, a substituted or unsubstituted alkyl group having 1 or more and 6 or less carbon atoms, and a substituted or unsubstituted aryl group having 6 or more and 12 or less carbon atoms, and
optionally R₁ to R₂₇ are each independently selected from the group consisting of a hydrogen atom, a deuterium atom, a methyl group, a CD₃ group, an isopropyl group, a tert-butyl group, C(CH₃)₂(C₂H₅), C(CH₃)(C₂H₅)₂, and a phenyl group.

3. The organic compound according to claim 1 or 2, wherein
in formula (1), Ar denotes a substituted or unsubstituted aryl group having 6 or more and 20 or less carbon atoms, and
optionally Ar denotes a substituted or unsubstituted aryl group having 6 or more and 12 or less carbon atoms.

4. The organic compound according to any one of claims 1 to 3, wherein
in formula (1), at least one of R₁, R₃, R₅, R₁₁, R₁₄, R₁₆, R₁₈, R₂₄, and R₂₇ is other than a hydrogen atom,
optionally at least one of R₂₄ and R₂₇ denotes a substituted or unsubstituted alkyl group, and
optionally R₂₄ and R₂₇ denote a methyl group.

5. The organic compound according to any one of claims 1 to 4, wherein
in formula (1), at least one of R₁, R₅, R₁₄, and R₁₈ denotes a substituted or unsubstituted alkyl group or a substituted or unsubstituted aryl group,
optionally at least one of R₁, R₅, R₁₄, and R₁₈ denotes a methyl group or a phenyl group, and
optionally at least one of R₃ and R₁₆ denotes a substituted or unsubstituted alkyl group.

6. An organic light-emitting element comprising:
a first electrode;
a second electrode (5); and
an organic compound layer (4) between the first electrode and the second electrode,
wherein the organic compound layer contains the organic compound according to any one of claims 1 to 5.

7. The organic light-emitting element according to claim 6, wherein
the organic compound layer includes a light-emitting layer, and
the light-emitting layer contains the organic compound.

8. The organic light-emitting element according to claim 7, wherein
the light-emitting layer further contains a first compound, and
the first compound has a higher lowest singlet excitation energy than the organic compound.

9. The organic light-emitting element according to claim 8, wherein the first compound comprises a hydrocarbon compound.

10. The organic light-emitting element according to claim 8, wherein
the light-emitting layer further contains a second compound, and
the second compound has a higher lowest singlet excitation energy than the organic compound and a lower lowest singlet excitation energy than the first compound.

11. A display apparatus comprising a plurality of pixels, wherein at least one of the plurality of pixels includes the organic light-emitting element according to any one of claims 6 to 10 and a transistor coupled to the organic light-emitting element.

12. A photoelectric conversion apparatus comprising:
an image pickup element configured to receive light; and
a display unit configured to display an image captured by the image pickup element,
wherein the display unit includes the organic light-emitting element according to any one of claims 6 to 10.

13. An image display apparatus comprising:
a display unit including the organic light-emitting element according to any one of claims 6 to 10; and
a housing in which the display unit is provided.

14. Electronic equipment comprising:
a display unit (1201) including the organic light-emitting element according to any one of claims 6 to 10;
a housing (1203) in which the display unit is provided; and
a communication unit that is provided in the housing and communicates with the outside.

15. A wearable device comprising:
a display unit including the organic light-emitting element according to any one of claims 6 to 10;
an optical system configured to condense light of the display unit; and
a controller configured to control display of the display unit.

16. A lighting apparatus comprising:
a light source (1402) including the organic light-emitting element according to any one of claims 6 to 10; and
a housing (1401) in which the light source is provided.

17. A movable body comprising:
a lamp including the organic light-emitting element according to any one of claims 6 to 10; and
a body provided with the lamp.
